# EUROPEAN PATENT APPLICATION

(11) **EP 0 955 383 A1**
(43) Date of publication of application: **10.11.1999**
(21) Application number: 99302321.7
(22) Date of filing: 25.03.1999
(51) Int. Cl.: C12Q 1/68, A61K 31/70, A61K 48/00

(54) **Method and marker for identification of pre-malignancy and malignancy and therapeutic intervention**

(30) Priority: 25.03.1998 US 79336 P
(71) Applicant: THE UNIVERSITY OF MANITOBA, Winnipeg, Manitoba R3E 0W3 (CA)
(72) Inventor: Johnston, James B., Winnepeg, Manitoba R3N 1M9 (CA); Mai, Sabine, Winnepeg, Manitoba R3N 1H9 (CA)
(74) Representative: BROOKES & MARTIN

(57) **Abstract**

There is provided a method for identifying pre-malignancy and malignancy states of a cell by detecting extrachromosomal gene amplification. Also provided is a marker for the identification of pre-malignancy and malignancy states of a cell comprising extrachromosomal gene amplification. A method of therapeutic intervention for cells having extrachromosomal gene amplification by therapeutically targeting the genes which have extrachromosomal amplification is provided.

## Description

### BACKGROUND OF THE INVENTION

### TECHNICAL FIELD

The present invention relates to methods and markers for identification of pre-malignancy and malignancy states utilizing extrachromosomal gene amplification. Further the present invention relates to the identification of specific genes which undergo extrachromosomal gene amplification and therapeutic interventions relating to their utility as therapeutic targets.

### BACKGROUND ART

The diagnosis of malignant conditions is approached from multiple directions as for example tissue biopsies, serum levels of specific markers (PSA for prostate as an example), mamography and the like. However, most of these methods do not identify pre-malignant cells where early diagnosis would significantly increase treatment potential. Further the identification of a malignant condition does not necessarily identify an underlying genetic abnormality which can be corrected utilizing gene therapy or suggest other points of therapeutic intervention.

Chronic lymphocytic leukemia (CLL), is the commonest leukemia, making up 30% of all cases (O'Brien, et al. 1995), However, the cause of this disease is unknown. The leukemia primarily affects elderly males and is characterized by the accumulation of morphologically mature-appearing B1-lymphocytes in peripheral blood, marrow, spleen and lymph nodes (O'Brien, et al., 1995). Prognosis in CLL is approximately assessed by Rai staging (Table I) and patient survival varies from 2 years (Rai III and IV) to >10 years (Rai 0) (Rai, et al., 1975). However, with each stage there is considerable variation in survival and patients may be further stratified according to the lymphocyte doubling time (Montserrat, et al., 1986). Patients with a short lymphocyte doubling time (<12 months) have a poorer survival rate than those with a longer doubling time (Montserrat, et al., 1986). At the present time, this disease is incurable but remissions may be obtained with alkylating agents, e.g., chlorambucil, or nucleoside analogs, e.g., fludarabine, but relapse and the eventual development of drug resistance is usually observed (O'Brien, et al., 1995).

The normal cellular counterpart of the CLL cell is in the mantle zone of the lymphoid follicle, and, like CLL cells, these lymphocytes are CD5+ B cells and have high levels of bcl-2 (Schena, et al., 1992). It is presumed that a small fraction of CLL cells are proliferating stem cells, possibly located in the lymphoid tissue or marrow, but the majority of cells are non-proliferating and accumulate most likely through defects in apoptosis.

The term genomic instability summarizes a variety of genomic alterations which include the loss or gain of chromosomes as well as genetic changes at the level of single genes, such as rearrangements, translocations, amplifications, deletions and point mutations, and has been considered to be a major driving force of multistep carcinogenesis (Nowell, 1976; Pienta et al., 1989; Temin, 1998; Solomon et al., 1991). Genomic integrity is maintained by checkpoint mechanisms; when cells suffer damage imposed by exposure to genotoxic drugs or microtubule toxins, the cell cycle is halted until the damage is repaired or apoptosis is initiated (for reviews, see Hartwell, 1992; Weinert and Lydall, 1993; Hartwell and Kastan, 1994).

Gene amplification represents one form of genomic instability in mammalian cells, although it may also occur as part of a normal developmental program in insects, amphibia, and lower organisms (Santelli, et al., 1991; Delikadis, et. al., 1989; Start, et. al., 1984). With one published exception (Prody, et al., 1989), gene amplification has not been observed in normal diploid cells (Lucke-Huhle, et al., 1989; Wright, et al., 1990; Tlsty, et al., 1990) and its presence indicates that these cells are genomically unstable, immortalized, transformed and/or tumorigenic. In mammalian cells lines and tumors, gene amplification has been described after drug selection (Stark, et al., 1993; Huang, et al., 1994; Huang, et al., 1994; Shah, et al., 1986), DNA damage (Lucke-Huhle, et al., 1989; Lucke-Huhle, et al., 1990; Yalkinoglu, et al., 1991) and as a result of c-Myc overexpression (Mai, et al., 1994; Denis, et al., 1991). Spontaneous gene amplification has also been reported (Johnston, et al., 1983). Gene amplification may occur in the presence of wildtype p53, but is facilitated by its absence (Yin, et al., 1992; Livingstone, et al., 1992); thus, gene amplification may involve both p53-dependent and -independent pathways (Van Der Bliek, et al., 1986; Zhou, et al., 1996). Gene amplifications often involves oncogenes, and more than 90% of these cases in patients involve c-myc where the degree of amplification correlates with the aggressiveness of tumor growth and poor prognosis (Schwab, et al., 1990).

c-Myc is a key regulator of growth, proliferation, differentiation, and development. Deregulation of the c-Myc oncoprotein has been reported in apoptosis, transformation, and in malignancies of lymphoid and non-lymphoid origin (Marcu, et al., 1992; Cole, et al., 1986). c-Myc plays a role in the modulation (Benevisty, et al., 1992; Bello-Fernandez, et al., 1993; Gaubatz, et al., 1994; Jansen-Durr, et al., 1993; Daksis, et al., 1994; Philipp, et al., 1994; Galaktinov, et al., 1996) and initiation of transcription (Roy, et al., 1993, Li, et al., 1994; Mai, et al., 1995). It is a short-lived nuclear oncoprotein (Cole, et al., 1986), which is strictly regulated during the cell cycle of normal diploid cells (Cole, et al., 1986; Heikkila, et al., 1987; Karn, et al., 1989). Increased half life of the protein is associated with immortalization and transformation (Marcu, et al., 1992; Cole, et al., 1986). The deregulation of c-Myc is a common feature in many tumors (Marcu, et al., 1992; Cole, et al., 1986), where it frequently is translocated (Stanton, et al., 1983; Potter, et al., 1992; Mai, et al., 1995; Marcu, et al., 1992; Cole, et al., 1986) and/or amplified and overexpressed (Marcu, et al., 1992; Cole, et al., 1986; Feo, et al., 1994; Alitalo, et al., 1985). In addition, the c-myc gene is often the site of proviral insertion (Marcu, et al, 1982; Cole, et al., 1986). Chromosomal aberrations involving c-myc are associated with a poor prognosis (Yokota, et al., 1986).

An amplified gene sequence is termed an 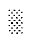 amplicon 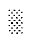 and may be chromosomal ( 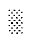 homogeneously staining regions 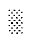 , HSR) or extrachromosomal ( 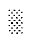 extrachromosomal elements 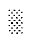 , Ees) . Extrachromosomal submicroscopic amplicons that replicate are termed 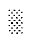 episomes 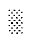 (250- 5,000 kb), and these can increase in size to be visible by light microscopy, at which point they are termed 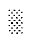 double minutes 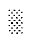 (>5,000 kb) (Stark, et al., 1993; Hahn, et al., 1993). A variety of mechanisms are involved in the production of gene amplification, and it appears likely that different mechanisms may be involved for different genes in the same cell or for the same gene in different cell types (Stark, et al., 1993; Stark, et al., 1989). The 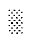 replication models 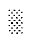 predict that a localized replication even would allow an isolated part of the chromosome to repeatedly replicate, i.e., onion-skin, double rolling circle or chromosome-spiral models, and these amplified areas may remain intrachromosomal or be released extrachromosomally. The second major group of mechanisms are the 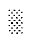 segregation-driven 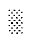 mechanisms, i.e., deletion-plus-episome and sister chromatid exchange models. The deletion-plus-episome theory predicts that deletion of a portion of chromosome produces Ees which can proliferate and be subsequently incorporated into random sites on a variety of chromosomes (Carroll, et al., 1988; Windle, et al., 1991).

Hamkalo et al, 1985 first showed using electron microscopy that the dihydrofolate reductase (DHFR) containing Ees in methorexate-resistant murine 3T3 cells are in circles, and numerous loops may be organized together in a rosette like structure. Similar findings have been observed by others in a variety of cell lines (Esnault, et al, 1994; Nonet, et al., 1993; Sen, et al., 1994; Schneider, et al., 1992; Cohen, et al., 1996; Cohen, et al., 1997). Esnault et al (Esnault, et al., 1994) isolated 630 kb Ees from a methorexate-resistant cell line and demonstrated that each of these Ees contained on DHFR gene. Transfection of the Ees into the methotrexate-sensitive parent cell line may confer methotrexate resistance. The adenosine deaminase containing Ees in cells grown in 2'-deoxycoformycin (Nonet, et al., 1993), c-myc containing Ees in HL-60 cells (Sen, et al., 1994) and N-myc containing Ees from neuroblastoma cells (Schneider, et al., 1992) have been isolated and cloned. In general, the genes remain intact, may be in a head-to-tail or head-to-head configuration (perhaps depending on the duration of time the Ees have existed) and appear to contain additional genomic loci to the studied gene. It has been suggested that these Ees may replicate and different sized molecules may develop through intra- or inter-molecular recombination (Esnault, et al., 1994). In addition, the number of Ees may increase in replicating cells, if they are unequally segregated to each daughter cell and provide the cell with a survival advantage (Stark, et al., 1993; Hahn, et al., 1993).

CLL has been studied by comparative genomic hybridization, which detects areas of chromosomal gene amplification or deletions (Bentz, et al., 1995). Abnormalities are detected in 70% of patients and one-third of these will have amplifications of all or part of chromosome 12 (Bentz, et al., 1995). Amplifications at 12q23-24, 12q13-22 and 12q13-15 have been observed by FISH in one patient with CLL (Merup, et al., 1997). To date, extrachromosomal gene amplification has only rarely been observed in CLL, and one patient has been described with extrachromosomal amplification of c-myc (Wang, et al., 1991).

While there are some protocols available for providing prognosis but within each stage there is considerable variation in survival. Therefore a better protocol for providing a prognosis and for making decisions on therapeutic strategies is needed.

### SUMMARY OF THE INVENTION

According to the present invention, there is provided a method for identifying pre-malignancy and malignancy states of a cell by detecting extrachromosomal gene amplification. Also provided is a marker for the identification of pre-malignancy and malignancy states of a cell comprising extrachromosomal gene amplification. A method of therapeutic intervention for cells having extrachromosomal gene amplification by therapeutically targeting the genes which have extrachromosomal amplification is provided.

### DESCRIPTION OF THE DRAWINGS

Other advantages of the present invention are readily appreciated as the same becomes better understood by reference to the following detailed description when considered in connection with the accompanying drawings wherein:

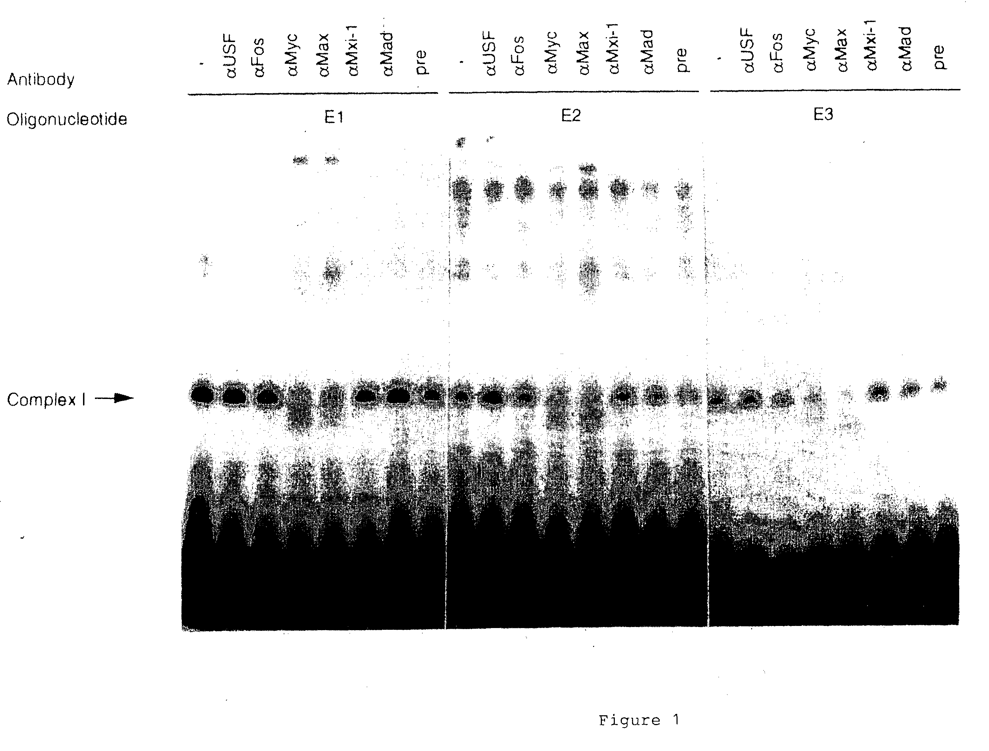

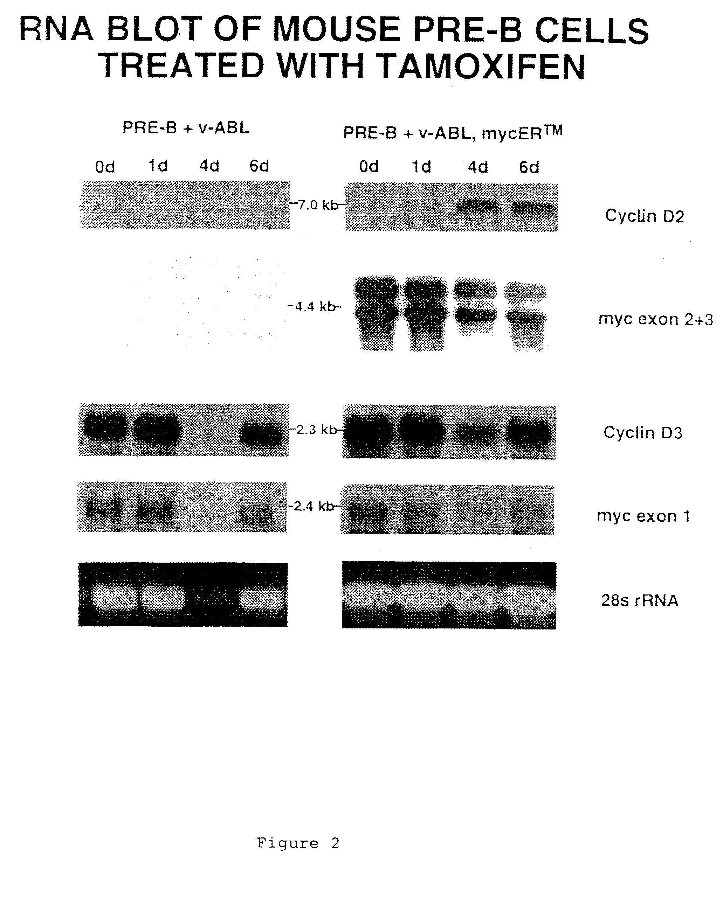

Figure 1 shows mobility (super-shift 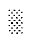 analysis using E1, E2, and E3 olignucleotides, 5 mg whole cell extracts of plasmacytoma MOPC 265 cells were incubated with the indicated antibodies or pre-immune serum (Pre) followed by the 32P-end-labeled oligonucleotides E1-E3; 1 mg of non-specific competitor DNA was used per reaction, the arrow points to the specific complex that does not form with bacterial protein alone; 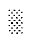 Supershifted 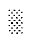 bands appear at the top of the anti-MYC and anti-MAX lanes, non-specific high-molecular weight bands appear in all lanes that use oligonucleotide E2, the antibody concentrations are indicated in Materials and Methods;
Figure 2 shows cyclin D2 expression after MYC activation; A. Northern blots of total RNA (15mg) from bulk cultures of mouse pre-B cells stably transduced with v-Abl (A-MuLV) or with v-Abl plus murine bcl-2 plus pBabePurorMycERTM, both cell lines were treated with 4HT for 0, 1, 4 and 6 days, as indicated, each blot was hybridized first with cyclin D2 cDNA and then sequentially with the other hybridization probes indicated along the right margin following stripping, sizes of major hybridizing bands are indicated between panels, ethidium-bromide-stained 28S ribosomal RNA bands are shown as loading controls; B. Western blots of 10 µg protein per lane, isolated from pre-B cells, with and without pBabePuroMycERTM, after different periods of stimulation with 4-HT, antibody specificity and size of detected protein bands are indicated between panels, actin probing of duplicate blots is shown as loading control; C. Northern analysis of total RNA (conditions as in A) from mouse fibroblasts (y2 cells) with and without stable integration of pBabePuroMyc-ERTM, after different numbers of days of stimulation with 4-HT, GAPDH hybridization is included as a loading control;

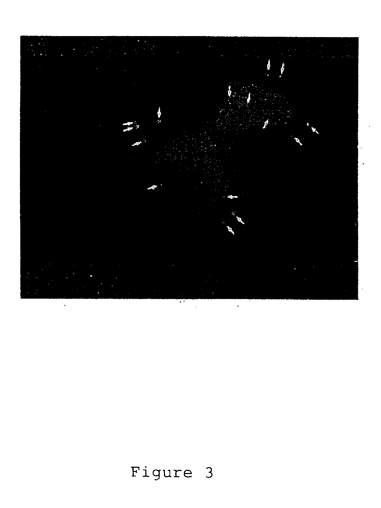

Figure 3 shows DHFR gene amplification in fully developed plasmacytoma. Arrows point to amplified DHFR sequences as detected by FISH. Nuclei are counterstained with DAPI (4', 6'-diamidino-2-phenylindole).

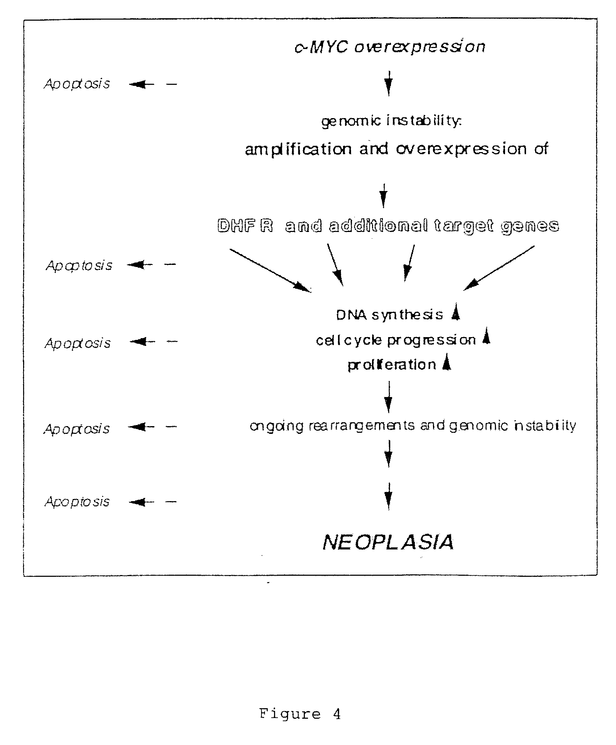

Figure 4 shows c-Myc deregulation and the initiation of genomic instability.

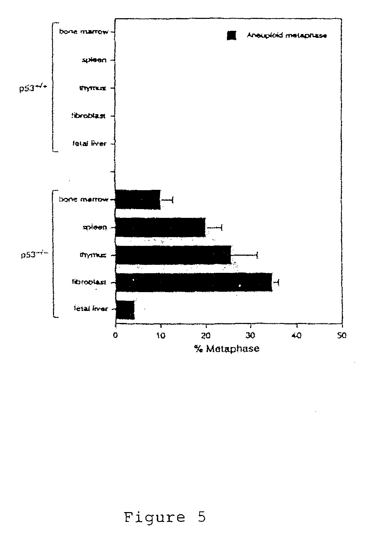

Figure 5 shows genomic instability in p53^{-/-} mice; Cytogenetic analysis of bone marrow, spleen, thumus-derived cells as well as of fibroblasts (passage 0) isolated from five p53^{-/-} and five parental p53^{+/+} (C57B1/6) mice, and of p53^{-/-} and p53^{+/+} fetal liver hematopoietic cells of 16 day old embryos;

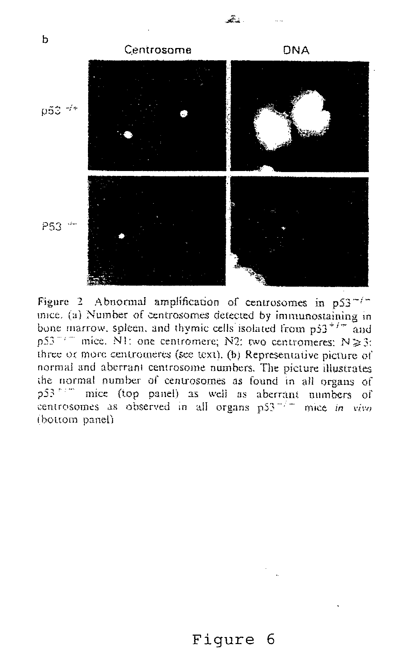

Figure 6 shows an abnormal amplification of centrosomes in p53^{-/-} mice; (a) Number of centrosomes detected by immunostaining in bone marrow, spleen, and thymic cells isolated from p53^{+/+} and p53^{-/-} mice. N1: one centromere; N2: two centromeres; N≥3: three or more centromeres (see text); (b) Representative picture of normal and aberrant centrosome numbers, the picture illustrates the normal number of centrosomes as found in all organs of p53^{+/+} mice (top panel) as well as aberrant numbers of centrosomes as observed in all organs p53^{-/-} mice *in vivo* (bottom panel);

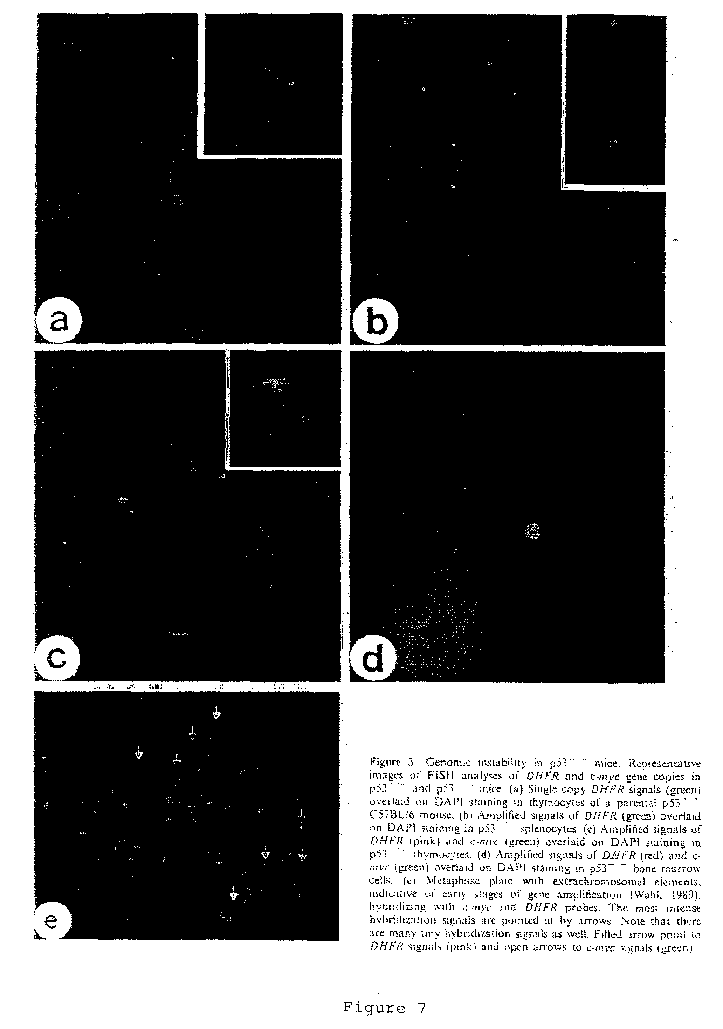

Figure 7 shows genomic instability in p53^{-/-} mice; Representative images of FISH analyses of DHFR and c-myc gene copies in p53^{+/+} and p53^{-/-} mice; (a) Single ocpy DHFR signals (green) overlaid on DAPI staining in thymocytes of a parental p53^{-/-} C57BL/6 mouse; (b) Amplified signals of DHFR (green) overlaid on DAPI staining in P53^{-/-} splenocytes; (c) Amplified signals of DHFR (pink) and c-myc (green) overlaid on DAPI staining in p53^{-/-} thymocytes; (d) Amplified signals of DHFR (red and c-myc (green) overlaid on DAPI staining in p53^{-/-} bone marrow cells; (e) Metaphase plate with extrachromosomal elements, indicative of early stages of gene amplification (Wahl, 1989), hybridizing with c-myc and DHFR probes, the most intense hybridization signals are pointed at by arrows, note that there are many tiny hybridization signals as well, filled arrow point to DHFR signals (pink) and open arrows to c-myc signals (green);

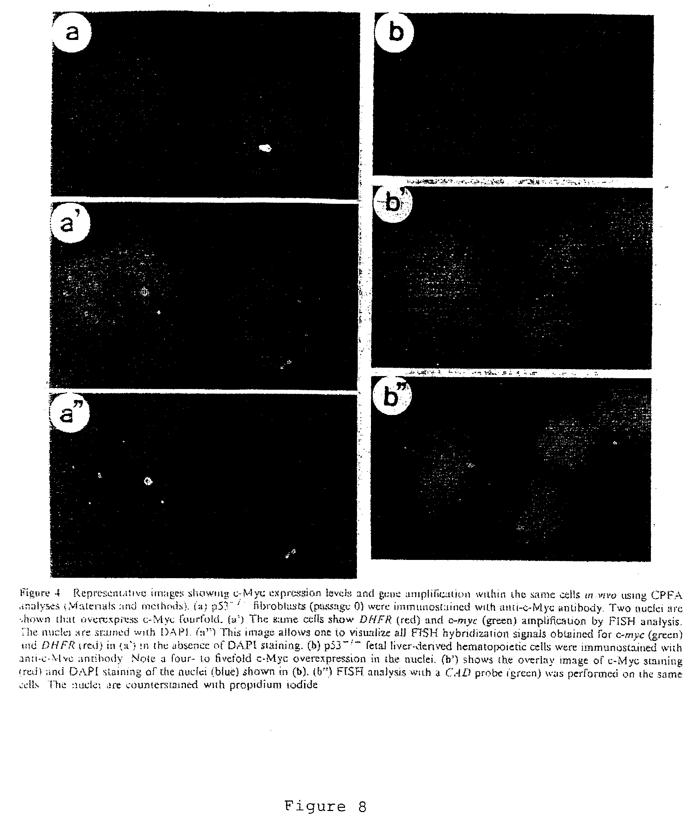

Figure 8 shows representative images showing c-Myc expression levels and gene amplification within the same cells *in vivo* using CPFA analyses (Materials and methods); (a) p53^{-/-} fibroblasts (passage 0) were immunostained with anti-c-Myc antibody, two nuclei are shown that overexpress c-Myc fourfold; (a') The same cells show DHFR (red) and c-myc (green) amplification by FISH analysis, the nuclei are stained with DAPI; (a 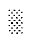 ) This image allows one to visualize all FISH hybridization signals obtained for c-myc (green) and DHFR (red) in (a') in the absence of DAPI staining; (b) p53^{-/-} fetal liver-derived hematopoietic cells were immunostained with anti-c-Myc antibody, note a four- to fivefold c-Myc overexpression in the nuclei; (b') shows the overlay image of c-Myc staining (red) and DAPI staining of the nuclei (blue) shown in (b); (b 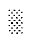 ) FISH analysis with a CAD probe (green) was performed on the same cells, the nuclei are counterstained with propidium iodide; and

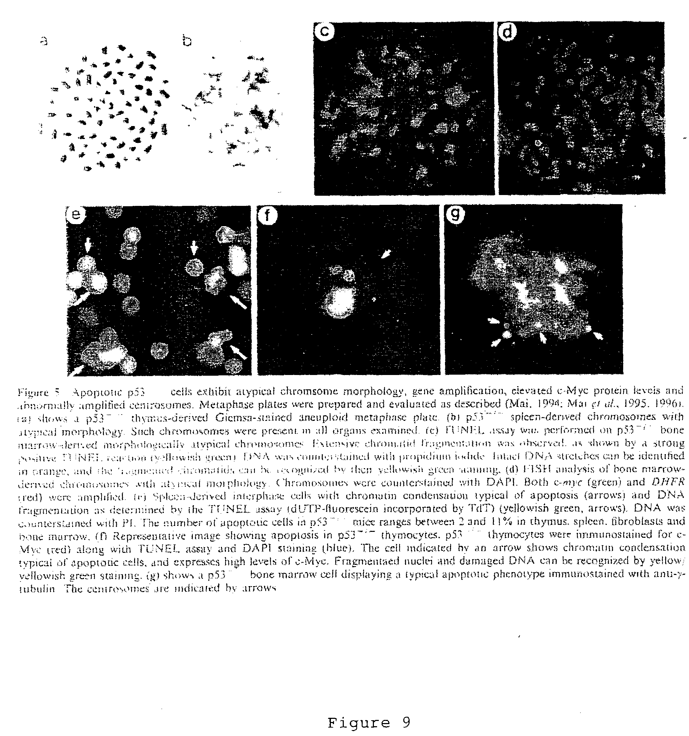

Figure 9 shows apoptotic p53^{-/-} cells exhibit atypical chromosome morphology, gene amplification, elevated c-Myc protein levels and abnormally amplified centrosomes, metaphase plates were prepared and evaluated as described (Mai, 1994; Mai 35 al., 1995, 1996); (a) shows a p53^{-/-} thymus-derived Giemsa-stained aneuploid metaphase plate; (b) p53^{-/-} spleen-derived chromosomes with atypical morphology, such chromosomes were present in all organs examined; (c) TUNEL assay was performed on p53^{-/-} bone marrow-derived morphologically atypical chromosomes, extensive chromatid fragmentation was observed, as shown by a strong positive TUNEL reaction (yellowish green); DNA was counterstained with propidium iodide, intact DNA stretches can be identified in orange, and the fragmented chromatids can be recognized by their yellowish green staining; (d) FISH analysis of bone marrow-derived chromosomes with atypical morphology, chromosomes were counterstained with DAPI, both c-myc (green) and DHFR (red) were amplified; (e) Spleen-derived interphase cells with chromatin condensation typical of apoptosis (arrows) and DNA fragmentation as determined by the TUNEL assay (dUTP-fluorescein incorporated by TdT) (yellowish green arrows), DNA was counterstained with PI, the number of apoptotic cells in P53^{-/-} mice ranges between 2 and 11% in thymus, spleen, fibroblasts and bone marrow; (f) Representative image showing apoptosis in p53^{-/-} thymocytes p53^{-/-} thymocytes were immunostained for c-Myc (red) along with TUNEL assay and DAPI staining (blue), the cell indicated by an arrow shows chromatin condensation yellowish green staining; (g) shows a p53^{-/-} bone marrow cell displaying a typical apoptotic phenotype immunostained with anti-tubulin, the centrosomes are indicated by arrows.

### DETAILED DESCRIPTION OF THE INVENTION

The present invention provides a method of identifying pre-malignant and malignant cells in cells and tissues. For example the present invention allows identification of micrometastasis, plasmacytomas, cervical cancer, head and neck cancer and CLL and other B-cell leukemias. More specifically, the method includes detecting the presence of extrachromosomal gene amplification in a cell. Additionally, a marker is disclosed which is used in the above method for identifying pre-malignancy and malignancy states in a cell by determining if extrachromosomal gene amplification of the marker is present.

The method of the present invention uses as a marker the presence of extrachromosomal gene amplification as the marker. Further, the present invention has unexpectedly determined that the specific genes that are amplified extrachromosomally are involved in initiation of tumorigenesis and therefore provide therapeutic targets. Therapeutic treatment including gene therapy as for example utilizing suicide genes targeted to the extrachomososmal elements or antisense therapy targeted to the identified genes may be utilized.

By extrachromosomal, it is meant a factor which exists, at least for a time, independent of the chromosome. Accordingly, the extrachromosomal factor is not a part of the chromosome, however these factors may be considered a genetic unit fully equal to those in the chromosomes.

Standard molecular biology techniques known in the art and not specifically described are generally followed as in Sambrook et al., *Molecular Cloning: A Laboratory Manual,* Cold Springs Harbor Laboratory, New York (1989, 1992), and in Ausubel et al., *Current Protocols in Molecular Biology,* John Wiley and Sons, Baltimore, Maryland (1989). Additionally, standard methods in immunology known in the art and not specifically described are generally followed as in Stites et al. (eds), *Basic and Clinical Immunology* (*8*^{*th*} *Edition),* Appleton & Lange, Norwalk, CT (1994) and Mishell and Shiigi (eds), *Selected Methods in Cellular Immunology,* W.H. Freeman and Co., New York (1980).

Cloning techniques are provided by the present invention. Immunoassays are also provided by the present invention. In general, ELISAs are the preferred immunoassays employed to assess a specimen. Both polyclonal and monoclonal antibodies can be used in the assays. The specific assay to be used can be determined by one skilled in the art.

Antibody production is provided by the present invention. Antibodies may be prepared against the immunogen, or any portion thereof, for example a synthetic peptide based on the sequence. As stated above, antibodies are used in assays and are therefore used in determining if the appropriate enzyme has been isolated. Antibodies can also be used for removing enzymes from red cell suspensions after enzymatic conversion. Immunogens can be used to produce antibodies by standard antibody production technology well known to those skilled in the art as described generally in Harlow and Lane, *Antibodies: A Laboratory Manual,* Cold Springs Harbor Laboratory, Cold Spring Harbor, NY, 1988 and Borrebaeck, *Antibody Engineering - A Practical Guide,* W.H. Freeman and Co., 1992. Antibody fragments may also be prepared from the antibodies and include Fab, F(ab')₂, and Fv by methods known to those skilled in the art.

Additional genes have been identified such as DHFR, c-MYC, immunoglobulin genes, anti-apoptosis genes, drug-resistance genes, that are amplified and play a role in the pathogenesis of other cancers such as plasmacytomas.

Inducible transfectants have been generated that allow the experimental overexpression of the c-Myc oncoprotein (Mai, 1994). The inducible overexpression of c-Myc is followed by the enhanced binding of the c-Myc/Max heterodimer (Mai, 1994; Mai, et al., 1996). Furthermore, the DHFR gene is amplified following the inducible overexpression of c-Myc and the increase in the c-Myc/Max heterodimer formation at the DHFR E-box motifs (Mai, 1994; Mai, et al., 1996). c-Myc overexpression thus affects the genomic stability of the DHFR locus.

The amplification of the DHFR gene occurs within three cell doublings and increases 1.8- to 4.2-fold during this time period. The amplification is locus-specific, since other loci are unaffected irrespective of c-Myc overexpression coincides with the elevated expression of the DHFR enzyme (Luecke-Huhle, et al., 1996). The amplification of the DHFR locus is transient if c-Myc overexpression is induced for a single time (Mai, et al., 1996). Constitutive c-Myc overexpression is associated with both the amplification and the rearrangement of the DHFR gene (Mai, et al., 1996). In agreement with these findings, it was observed that the prolonged induction of c-Myc in inducible lines is accompanied with ongoing amplification and rearrangements of the DHFR gene (Mai, et al., 1996). Moreover, the prolonged induction of c-Myc overexpression induces a significant increase in the formation of telomere-centromere-fusions and of extrachromosomal elements (Mai, et al., 1996).

Recent studies have also shown that DHFR gene amplification occurs in association with c-Myc upregulation in p53 deficient mice *in vivo* (Fukasawa, et al., 1997). However, using p53-deficient mice as an experimental model does not resolve the question as to whether c-Myc overexpression precedes the amplification of the DHFR gene. The present work was initiated to determine whether the locus-specific amplification of the DHFR gene occurred as a result of induced c-Myc overexpression *in vivo.*

To this end, an animal model of c-Myc-dependent neoplasia was examined, the mouse plasmacytoma. Plasmacytomagenesis is the neoplastic development of mouse B lineage cells (Potter et al., 1992). Four criteria generally define a plasmacytoma (PCT) cell: i) It has well developed Golgi and is rich in endoplasmic reticulum; ii) It predominantly secretes IgG and IgA. IgM and IgD were reported in a few cases; iii) It commonly displays a translocation between c-Myc (chromosome 15) and immunoglobulin (Ig) loci (chromosomes 12, 6, 16), and iv) c-Myc is constitutively expressed due to the juxtaposition of myc and Ig loci. PCTs can be experimentally induced in the mouse, with a strain specific predisposition (Potter, et al., 1992). Balb/c and NZB mice strains are susceptible to PCTgenesis, whereas DBA/2, C57BL/6 and C3H mice are not (<5% develop paraffin oil induced PCTs). Susceptibility loci are located on chromosome 4 and 1, respectively (Mock, et al., 1993; Potter, et al., 1994). The experimental induction of PCTs is achieved with paraffin oils and pristane or plastic implants; in rare cases, plasmacytomas may arise spontaneously (Potter, et al., 1992; Silva, et al., 1997).

To examine whether c-Myc-dependent DHFR gene amplification occurred *in vivo*, PCT-susceptible Balb/c mice were analyzed following the i.p. injection of pristane that leads to the induction of PCTs in these mice (Potter, et al., 1992). The c-Myc overexpression associated amplification of the DHFR gene in pristane-injected Balb/c mice is shown. This amplification does not involve the polyploidization of the DHFR carrying chromosome 13 and is predominantly observed on extrachromosomal elements.

The focus of the experimental work herein has been on MYC-dependent genomic instability. The c-Myc overexpression is associated with the non-random amplification and rearrangement of the dihydrofolate reductase (Dhfr, ref. Mai 1994 and Mai et al., 1996) gene and the gene encoding the R2 subunit of ribonucleotide reductase, RNR2, but not the RNR1 gene (T.I. Kuschak et al., submitted).

In the present study, evidence is shown of MYC-dependent amplification of the cyclin D2 locus and attendant increased cyclin D2 gene products. These findings link c-Myc overexpression and cell cycle regulation for the first time at the level of genomic instability of this G1 cyclin. Based on these findings, a model of MYC-dependent genomic instability and neoplasia is established.

Constitutive Myc expression is a key element in the induction of mouse plasmacytomas and human Burkitt lymphomas. The mechanism through which Myc over-expression contributes to this and other forms of carcinogenesis remains elusive. One consequence of Myc overexpression is a shortening of the G1 phase of cell cycle, these elements that regulated this phase were studied and cloned cDNAs for the 3 mouse D cyclins. Northern blots of RNAs from B-cell tumors showed that plasmacytomas have not only abundant c-Myc transcript but also high levels of cyclin D2 transcripts. In the genomic clone a 4 CACGTG 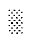 EMS (E-box Myc Site) motifs 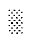 was found upstream of the cyclin D2 coding region. These EMS motifs bind Myc/Max heterodimers, suggesting that constitutive Myc expression may cause cyclin D2 overexpression. Cyclin D2 DNA and mRNA were examined in established mouse and human tumors that expressed high levels of Myc. Also examined was the substantial amplification of the cyclin D2 gene by Southern blotting and by FISH.

To follow the Myc/cyclin D2 connection *in vitro,* Myc activity was upregulated in two cell lines using an expression vector making a Myc-ER chimera that is activated by 4-hydroxytamoxifen (4HT). In mouse pre-B cells and fibroblasts that bear Myc-ERTM, cyclin D2 mRNA rose after 3-4 days of 4HT activation of Myc. Simultaneously, evidence of amplification in the form of extrachromosomal elements was seen by FISH. Thus, one important action of Myc seems to be the induction of gene amplification, a form of genomic instability. The associated increase in cyclin D2 gene products, apparently due to the amplification, rather than upregulated transcription, can contribute to uncontrolled growth.

The dihydrofolate reductase (DHFR) gene is a target of c-Myc in genomic instability. The induced overexpression of c-Myc in cell lines is followed by the amplification and rearrangement of the DHFR gene. Furthermore, the constitutive upregulation of c-Myc protein coincides with genomic instability of the DHFR gene in lymphoid, non-lymphoid and in tumor lines. The amplification of the DHFR gene is locus-specific and independent of species origins. The question has been addressed whether inducible deregulation of c-Myc is followed by DHFR gene amplification *in vivo*. Therefore, the DHFR gene is a target of c-Myc-dependent neoplasia *in vivo* and plays a role in genomic instability during the initiation of neoplastic transformation.

It has also been established that the loss of p53 tumor suppressor functions results in genetic instability, characteristically associated with changes in chromosome ploidy and gene amplification. *In vivo,* cells from various organs of 4 to 6-week old p53-nullizygous (p53^{-/-}) mice display aneuploidy and frequent gene amplification as well as evidence of apoptosis. Regardless of tissue types, many p53^{-/-} cells contain multiple centrosomes and abnormally formed mitotic spindles. Thus, chromosome instability *in* vivo may be associated with abnormal centrosome amplification. Moreover, a significant increase in the number of cells overexpressing c-Myc in p53^{-/-} mice is observed. Consistent with previous studies showing that c-Myc overexpression is associated with gene amplification *in vitro,* may of the p53^{-/-} cells exhibited, in the same cell, c-Myc overexpression and amplified c-myc, dihydrofolate reductase (DHFR), and carbamoyl-phosphate syntehtase-asparate transcarbamoyl-dihydroorotase (CAD) genes. Furthermore, apoptosis was frequently observed in cells isolated from p53^{-/-} mice. The apoptotic cells contained abnormally amplified centrosomes, displayed aneuploidy, high levels of c-Myc expression, as well as gene amplification. These results indicate that a high number of aberrant cells is eliminated by p53-independent pathways *in vitro.*

In another embodiment of the present invention there is provided a kit for identifying pre-malignancy and malignant states of a cell. The kit contains a device for detecting extrachromosomal gene amplification. More specifically, the kit will detect extrachromosomal gene amplification of genes such as DHFR, C-Myc, immunoglobulin genes, anti-apoptosis genes and drug-resistance genes. The process for detecting the gene amplification will use a combined protein and FISH analysis. Such analysis will include a detecting gene amplification using quantitative fluorescence immunohistochemistry. The kit may also include items for therapeutic intervention of cells having such extrachromosomal gene amplification. Such therapeutic intervention will include therapeutically targeting the genes which have extrachromosomal amplification. Such therapy may include gene therapy such as suicide genes which are targeted toward extrachromosomal elements or antisense therapy targeted to the identified gene.

The methods used with and the utility of the present invention can be shown by the following non-limiting examples and accompanying figures and incorporated by reference in their entirety.

The above discussion provides a factual basis for the use of markers for the identification of pre-malignancy and malignancy of cells. The methods used with and the utility of the present invention can be shown by the following non-limiting examples and accompanying figures.

### EXAMPLES

### GENERAL METHODS:

**General methods in molecular biology**: Standard molecular biology techniques known in the art and not specifically described are generally followed as in Sambrook et al., *Molecular Cloning: A Laboratory Manual,* Cold Springs Harbor Laboratory, New York (1989, 1992), and in Ausubel et al., *Current Protocols in Molecular Biology,* John Wiley and Sons, Baltimore, Maryland (1989). Polymerase chain reaction (PCR) is carried out generally as in *PCR Protocols: A Guide To Methods And Applications,* Academic Press, San Diego, CA (1990). Reactions and manipulations involving other nucleic acid techniques, unless stated otherwise, are performed as generally described in Sambrook et al., 1989, *Molecular Cloning: A Laboratory Manual,* Cold Spring Harbor Laboratory Press, and methodology as set forth in United States patents 4,666,828; 4,683,202; 4,801,531; 5,192,659 and 5,272,057 and incorporated herein by reference. In-situ (In-cell) PCR in combination with Flow Cytometry can be used for detection of cells containing specific DNA and mRNA sequences (Testoni et al, 1996, Blood 87:3822.)

**General methods in immunology:** Standard methods in immunology known in the art and not specifically described are generally followed as in Stites et al.(eds), Basic and Clinical Immunology (8th Edition), Appleton & Lange, Norwalk, CT (1994) and Mishell and Shiigi (eds), Selected Methods in Cellular Immunology, W.H. Freeman and Co., New York (1980).

Immumoassays In general, ELISAs are the preferred immunoassays employed to assess a specimen. ELISA assays are well known to those skilled in the art. Both polyclonal and monoclonal antibodies can be used in the assays. here appropriate other immunoassays, such as radioimmunoassays (RIA) can be used as are known to those in the art. Available immunoassays are extensively described in the patent and scientific literature. See, for example, United States patents 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521 as well as Sambrook et al, *Molecular Cloning: A Laboratory Manual,* Cold Springs Harbor, New York, 1989.

### Transgenic and Knockout Methods

The present invention may provide for transgenic gene and polymorphic gene animal and cellular (cell lines) models as well as for knockout models. These models are constructed using standard methods known in the art and as set forth in United States Patents 5,487,992, 5,464,764, 5,387,742, 5,360,735, 5,347,075, 5,298,422, 5,288,846, 5,221,778, 5,175,385, 5,175,384,5,175,383, 4,736,866 as well as Burke and Olson (1991), Capecchi (1989), Davies et al. (1992), Dickinson et al. (1993), Duff and Lincoln (1995), Huxley et al. (1991), Jakobovits et al. (1993), Lamb et al. (1993), Pearson and Choi (1993), Rothstein (1991), Schedl et al. (1993), Strauss et al. (1993). Further, patent applications WO 94/23049, WO 93/14200, WO 94/06908, WO 94/28123 also provide information.

**For gene therapy:** By gene therapy as used herein refers to the transfer of genetic material (e.g DNA or RNA) of interest into a host to treat or prevent a genetic or acquired disease or condition phenotype. The genetic material of interest encodes a product (e.g. a protein, polypeptide, peptide or functional RNA, antisense) whose production *in vivo* is desired. For example, the genetic material of interest can encode a hormone, receptor, enzyme, polypeptide or peptide or antisense sequence of therapeutic value. For a review see, in general, the text "Gene Therapy" (Advances in Pharmacology 40, Academic Press, 1997).

Two basic approaches to gene therapy have evolved: (1) *ex vivo* and (2) *in vivo* gene therapy. In *ex vivo* gene therapy cells are removed from a patient, and while being cultured are treated *in vitro.* Generally, a functional genetic sequence is introduced into the cell via an appropriate gene delivery vehicle/method (transfection, transduction, homologous recombination, etc.) and an expression system as needed and then the modified cells are expanded in culture and returned to the host/patient. These genetically reimplanted cells have been shown to have the transfected genetic sequence expressed *in situ.*

In *in vivo* gene therapy, target cells are not removed from the subject rather the genetic sequence to be transferred is introduced into the cells of the recipient organism *in situ,* that is within the recipient. Alternatively, if the host gene is defective, the gene is repaired *in situ* (Culver, 1998). These genetically altered cells have been shown to express the transfected gene sequence *in situ.*

The gene expression vehicle is capable of delivery/transfer of heterologous nucleic acid into a host cell. The expression vehicle may include elements to control targeting, expression and transcription of the nucleic acid in a cell selective manner as is known in the art. It should be noted that often the 5'UTR and/or 3'UTR of the gene may be replaced by the 5'UTR and/or 3'UTR of the expression vehicle. Therefore as used herein the expression vehicle may, as needed, not include the 5'UTR and/or 3'UTR of the gene of interest and only include the specific amino acid coding region of the gene of interest.

The expression vehicle can include a promotor for controlling transcription of the heterologous material and can be either a constitutive or inducible promotor to allow selective transcription. Enhancers that may be required to obtain necessary transcription levels can optionally be included. Enhancers are generally any non-translated DNA sequence which works contiguously with the coding sequence (in cis) to change the basal transcription level dictated by the promoter. The expression vehicle can also include a selection gene as described herein below.

Vectors can be introduced into cells or tissues by any one of a variety of known methods within the art. Such methods can be found generally described in Sambrook et al., *Molecular Cloning: A Laboratory Manual,* Cold Springs Harbor Laboratory, New York (1989, 1992), in Ausubel et al., *Current Protocols in Molecular Biology,* John Wiley and Sons, Baltimore, Maryland (1989), Chang et al., *Somatic Gene Therapy,* CRC Press, Ann Arbor, MI (1995), Vega et al., *Gene Targeting,* CRC Press, Ann Arbor, MI (1995), *Vectors: A Survey of Molecular Cloning Vectors and Their Uses,* Butterworths, Boston MA (1988) and Gilboa et al (1986) and include, for example, stable or transient transfection, lipofection, electroporation and infection with recombinant viral vectors. In addition, see United States patent 4,866,042 for vectors involving the central nervous system and also United States patents 5,464,764 and 5,487,992 for positive-negative selection methods.

Introduction of nucleic acids by infection offers several advantages over the other listed methods. Higher efficiency can be obtained due to their infectious nature. Moreover, viruses are very specialized and typically infect and propagate in specific cell types. Thus, their natural specificity can be used to target the vectors to specific cell types *in vivo* or within a tissue or mixed culture of cells. Viral vectors can also be modified with specific receptors or ligands to alter target specificity through receptor mediated events.

A specific example of DNA viral vector for introducing and expressing recombinant sequences is the adenovirus derived vector Adenop53TK. This vector expresses a herpes virus thymidine kinase (TK) gene for either positive or negative selection and an expression cassette for desired recombinant sequences. This vector can be used to infect cells that have an adenovirus receptor which includes most cancers of epithelial origin as well as others. This vector as well as others that exhibit similar desired functions can be used to treat a mixed population of cells and can include, for example, an *in vitro* or *ex vivo* culture of cells, a tissue or a human subject.

Additional features can be added to the vector to ensure its safety and/or enhance its therapeutic efficacy. Such features include, for example, markers that can be used to negatively select against cells infected with the recombinant virus. An example of such a negative selection marker is the TK gene described above that confers sensitivity to the antibiotic gancyclovir. Negative selection is therefore a means by which infection can be controlled because it provides inducible suicide through the addition of antibiotic. Such protection ensures that if, for example, mutations arise that produce altered forms of the viral vector or recombinant sequence, cellular transformation will not occur.

Features that limit expression to particular cell types can also be included. Such features include, for example, promoter and regulatory elements that are specific for the desired cell type.

In addition, recombinant viral vectors are useful for *in* vivo expression of a desired nucleic acid because they offer advantages such as lateral infection and targeting specificity. Lateral infection is inherent in the life cycle of, for example, retrovirus and is the process by which a single infected cell produces many progeny virions that bud off and infect neighboring cells. The result is that a large area becomes rapidly infected, most of which was not initially infected by the original viral particles. This is in contrast to vertical-type of infection in which the infectious agent spreads only through daughter progeny. Viral vectors can also be produced that are unable to spread laterally. This characteristic can be useful if the desired purpose is to introduce a specified gene into only a localized number of targeted cells.

As described above, viruses are very specialized infectious agents that have evolved, in many cases, to elude host defense mechanisms. Typically, viruses infect and propagate in specific cell types. The targeting specificity of viral vectors utilizes its natural specificity to specifically target predetermined cell types and thereby introduce a recombinant gene into the infected cell. The vector to be used in the methods of the invention will depend on desired cell type to be targeted and are known to those skilled in the art. For example, if breast cancer is to be treated then a vector specific for such epithelial cells would be used. Likewise, if diseases or pathological conditions of the hematopoietic system are to be treated, then a viral vector that is specific for blood cells and their precursors, preferably for the specific type of hematopoietic cell, would be used.

Retroviral vectors can be constructed to function either as infectious particles or to undergo only a single initial round of infection. In the former case, the genome of the virus is modified so that it maintains all the necessary genes, regulatory sequences and packaging signals to synthesize new viral proteins and RNA. Once these molecules are synthesized, the host cell packages the RNA into new viral particles which are capable of undergoing further rounds of infection. The vector's genome is also engineered to encode and express the desired recombinant gene. In the case of non-infectious viral vectors, the vector genome is usually mutated to destroy the viral packaging signal that is required to encapsulate the RNA into viral particles. Without such a signal, any particles that are formed will not contain a genome and therefore cannot proceed through subsequent rounds of infection. The specific type of vector will depend upon the intended application. The actual vectors are also known and readily available within the art or can be constructed by one skilled in the art using well-known methodology.

The recombinant vector can be administered in several ways. If viral vectors are used, for example, the procedure can take advantage of their target specificity and consequently, do not have to be administered locally at the diseased site. However, local administration can provide a quicker and more effective treatment, administration can also be performed by, for example, intravenous or subcutaneous injection into the subject. Injection of the viral vectors into a spinal fluid can also be used as a mode of administration, especially in the case of neuro-degenerative diseases. Following injection, the viral vectors will circulate until they recognize host cells with the appropriate target specificity for infection.

An alternate mode of administration can be by direct inoculation locally at the site of the disease or pathological condition or by inoculation into the vascular system supplying the site with nutrients or into the spinal fluid. Local administration is advantageous because there is no dilution effect and, therefore, a smaller dose is required to achieve expression in a majority of the targeted cells. Additionally, local inoculation can alleviate the targeting requirement required with other forms of administration since a vector can be used that infects all cells in the inoculated area. If expression is desired in only a specific subset of cells within the inoculated area, then promoter and regulatory elements that are specific for the desired subset can be used to accomplish this goal. Such non-targeting vectors can be, for example, viral vectors, viral genome, plasmids, phagemids and the like. Transfection vehicles such as liposomes can also be used to introduce the non-viral vectors described above into recipient cells within the inoculated area. Such transfection vehicles are known by one skilled within the art.

Antisense Therapy: Many reviews have covered the main aspects of antisense (AS) technology and its enormous therapeutic potential (Wright and Anazodo, 1995). There are reviews on the chemical (Crooke, 1995; Uhlmann et al, 1990), cellular (Wagner, 1994) and therapeutic (Hanania, *et al,* 1995; Scanlon, *et al,* 1995; Gewirtz, 1993) aspects of this rapidly developing technology. Within a relatively short time, ample information has accumulated about the *in vitro* use of AS nucleotide sequences in cultured primary cells and cell lines as well as for *in vivo* administration of such nucleotide sequences for suppressing specific processes and changing body functions in a transient manner. Further, enough experience is now available *in vitro* and *in vivo* in animal models and human clinical trials to predict human efficacy.

Antisense intervention in the expression of specific genes can be achieved by the use of synthetic AS oligonucleotide sequences (for recent reports see Lefebvre-d'Hellencourt *et al,* 1995; Agrawal, 1996; Lev-Lehman *et al,* 1997). AS oligonucleotide sequences may be short sequences of DNA, typically 15-30 mer but may be as small as 7 mer (Wagner et al, 1996), designed to complement a target mRNA of interest and form an RNA:AS duplex. This duplex formation can prevent processing, splicing, transport or translation of the relevant mRNA. Moreover, certain AS nucleotide sequences can elicit cellular RNase H activity when hybridized with their target mRNA, resulting in mRNA degradation (Calabretta *et al,* 1996). In that case, RNase H will cleave the RNA component of the duplex and can potentially release the AS to further hybridize with additional molecules of the target RNA. An additional mode of action results from the interaction of AS with genomic DNA to form a triple helix which may be transcriptionally inactive.

Phosphorothioate antisense oligonucleotides do not normally show significant toxicity at concentrations that are effective and exhibit sufficient pharmacodynamic half-lives in animals (Agarwal *et al.,* 1996) and are nuclease resistant. Antisense induced loss-of-function phenotypes related with cellular development were shown for the glial fibrillary acidic protein (GFAP), for the establishment of tectal plate formation in chick (Galileo *et al.,* 1991) and for the N-myc protein, responsible for the maintenance of cellular heterogeneity in neuroectodermal cultures (ephithelial vs. neuroblastic cells, which differ in their colony forming abilities, tumorigenicity and adherence) (Rosolen *et al.,* 1990; Whitesell *et al,* 1991). Antisense oligonucleotide inhibition of basic fibroblast growth factor (bFgF), having mitogenic and angiogenic properties, suppressed 80% of growth in glioma cells (Morrison, 1991) in a saturable and specific manner. Being hydrophobic, antisense oligonucleotides interact well with phospholipid membranes (Akhter *et al.,* 1991). Following their interaction with the cellular plasma membrane, they are actively (or passively) transported into living cells (Loke *et al.,* 1989), in a saturable mechanism predicted to involve specific receptors (Yakubov *et al.,* 1989).

Instead of an antisense sequence as discussed herein above, ribozymes may be utilized. This is particularly necessary in cases where antisense therapy is limited by stoichiometric considerations (Sarver et al., 1990, Gene Regulation and Aids, pp. 305-325). Ribozymes can then be used that will target the same sequence. Ribozymes are RNA molecules that possess RNA catalytic ability (see Cech for review) that cleave a specific site in a target RNA. The number of RNA molecules that are cleaved by a ribozyme is greater than the number predicted by stoichiometry. (Hampel and Tritz, 1989; Uhlenbeck, 1987).

Ribozymes catalyze the phosphodiester bond cleavage of RNA. Several ribozyme structural families have been identified including Group I introns, RNase P, the hepatitis delta virus ribozyme, hammerhead ribozymes and the hairpin ribozyme originally derived from the negative strand of the tobacco ringspot virus satellite RNA (sTRSV) (Sullivan, 1994; U.S. Patent No. 5,225,347, columns 4-5). The latter two families are derived from viroids and virusoids, in which the ribozyme is believed to separate monomers from oligomers created during rolling circle replication (Symons, 1989 and 1992). Hammerhead and hairpin ribozyme motifs are most commonly adapted for trans-cleavage of mRNAs for gene therapy (Sullivan, 1994). The ribozyme type utilized in the present invention is selected as is known in the art. Hairpin ribozymes are now in clinical trial and are the preferred type. In general the ribozyme is from 30-100 nucleotides in length.

The present invention provides pharmaceutical compositions for the delivery of the oligonucleotides of the present invention. The pharmaceutical compositions contain active ingredients as described herein and a pharmaceutically suitable carrier or diluent. The compositions can be administered orally, subcutaneously or parenterally including intravenous, intraarterial, intramuscular, intraperitoneally, and intranasal administration as well as intrathecal and infusion techniques as required by the cells being treated. For delivery within the CNS intrathecal delivery can be used with for example an Ommaya reservoir or other methods known in the art. The pharmaceutically acceptable carriers, diluents, adjuvants and vehicles as well as implant carriers generally refer to inert, non-toxic solid or liquid fillers, diluents or encapsulating material not reacting with the active ingredients of the invention. Implants of the compounds are also useful. In general the pharmaceutical compositions are sterile.

Modifications or analogues of nucleotides can be introduced to improve the therapeutic properties of the nucleotides. Improved properties include increased nuclease resistance and/or increased ability to permeate cell membranes.

Nuclease resistance, where needed, is provided by any method known in the art that does not interfere with biological activity of the antisense oligodeoxynucleotides and/or ribozymes as needed for the method of use and delivery (Iyer et al., 1990; Eckstein, 1985; Spitzer and Eckstein, 1988; Woolf et al., 1990; Shaw et al., 1991). Modifications that can be made to oligonucleotides in order to enhance nuclease resistance include modifying the phophorous or oxygen heteroatom in the phosphate backbone. These include preparing methyl phosphonates, phosphorothioates, phosphorodithioates and morpholino oligomers. In one embodiment it is provided by having phosphorothioate bonds linking between the four to six 33-terminus nucleotide bases. Alternatively, phosphorothioate bonds link all the nucleotide bases. Other modifications known in the art may be used where the biological activity is retained, but the stability to nucleases is substantially increased.

The present invention also includes all analogues of, or modifications to, an oligonucleotide of the invention that does not substantially affect the function of the oligonucleotide. The nucleotides can be selected from naturally occurring or synthetic modified bases. Naturally occurring bases include adenine, guanine, cytosine, thymine and uracil. Modified bases of the oligonucleotides include xanthine, hypoxanthine, 2-aminoadenine, 6-methyl, 2-propyl and other alkyl adenines, 5-halo uracil, 5-halo cytosine, 6-aza cytosine and 6-aza thymine, psuedo uracil, 4-thiuracil, 8-halo adenine, 8-aminoadenine, 8-thiol adenine, 8-thiolalkyl adenines, 8-hydroxyl adenine and other 8-substituted adenines, 8-halo guanines, 8-amino guanine, 8-thiol guanine, 8-thioalkyl guanines, 8-hydroxyl guanine and other substituted guanines, other aza and deaza adenines, other aza and deaza guanines, 5-trifluoromethyl uracil and 5-trifluoro cytosine.

In addition, analogues of nucleotides can be prepared wherein the structure of the nucleotide is fundamentally altered and that are better suited as therapeutic or experimental reagents. An example of a nucleotide analogue is a peptide nucleic acid (PNA) wherein the deoxyribose (or ribose) phosphate backbone in DNA (or RNA) is replaced with a polyamide backbone which is similar to that found in peptides. PNA analogues have been shown to be resistant to degradation by enzymes and to have extended lives *in vivo* and *in vitro.* Further, PNAs have been shown to bind stronger to a complementary DNA sequence than a DNA molecule. This observation is attributed to the lack of charge repulsion between the PNA strand and the DNA strand. Other modifications that can be made to oligonucleotides include polymer backbones, cyclic backbones, or acyclic backbones.

The active ingredients include oligonucleotides that are nuclease resistant needed for the practice of the invention or a fragment thereof shown to have the same effect targeted against the appropriate sequence(s) and/or ribozymes. Combinations of the active ingredients can be used.

The antisense oligonucleotides (and/or ribozymes) of the present invention can be synthesized by any method known in the art for ribonucleic or deoxyribonucleic nucleotides. For example, an Applied Biosystems 380B DNA synthesizer can be used. When fragments are used, two or more such sequences can be synthesized and linked together for use in the present invention.

The nucleotide sequences of the present invention can be delivered either directly or with viral or non-viral vectors. When delivered directly the sequences are generally rendered nuclease resistant. Alternatively the sequences can be incorporated into expression cassettes or constructs such that the sequence is expressed in the cell. Generally the construct contains the proper regulatory sequence or promotor to allow the sequence to be expressed in the targeted cell.

**Delivery of gene products/therapeutics (compound):** The compound of the present invention is administered and dosed in accordance with good medical practice, taking into account the clinical condition of the individual patient, the site and method of administration, scheduling of administration, patient age, sex, body weight and other factors known to medical practitioners. The pharmaceutically "effective amount" for purposes herein is thus determined by such considerations as are known in the art. The amount must be effective to achieve improvement including but not limited to improved survival rate or more rapid recovery, or improvement or elimination of symptoms and other indicators as are selected as appropriate measures by those skilled in the art.

### Example 1

*Mice.* Balb/c mice were obtained from Dentistry (University of Manitoba) and kept according to the international standards of Central Animal Care. All experiments performed were in accordance with the approved animal protocol (95-441). An age group of 406 week old Balb/c mice (20 mice each) received i.p. injections of 0.5 ml pristane (2,6,10,14-tetramethylpentadecane (Sigma)) or LPS (lipopolysaccharide (Sigma)) at 125 µg/ml (20 mice each). At the time points indicated in the text, samples were taken from the peritoneal cavity of the experimental mice. To this end, the mice were anesthesized using avertin (2,2,2-tribromoethanol (Aldrich)). 1 g avertin was dissolved in 0.5 ml liquid tertiary amyl alcohol (Aldrich). 0.5 ml of this solution was diluted with 39.5 ml warm (37°C) phosphate buffered saline (PBS) and used to anesthesize the mice. The average dose per mouse was dependent on the body weight of the animal; a mouse of 20g received 0.35 ml. After anesthesia, peritoneal cavity cells were collected with 8-10 ml of 37°C prewarmed sterile culture medium that did not contain fetal calf serum.

Cells directly isolated from the experimental mice were immobilized on microscopic slides using a cytospin centrifuge. Fluorescent immunohistochemistry was carried out using a mouse anti-c-Myc antibody (3C7, reef. 13) at 20 mg per slide, followed by a goat anti-mouse IgG-Texas Red antibody (Southern Biotechnology Associates, Inc., USA) at 10 µg per slide. The fluorescence intensity was quantitated using the Multiprobe 1.1E software (Signal Analytics, USA) (Mai, et al., 1996). One hundred to three hundred cells were evaluated per sample.

Fluorescent *in situ* hybridization (FISH) was used to determine gene copy numbers on a single cell level. The DHFR probe used for hybridization as well as the hybridization conditions and the image analysis have been described earlier (Mai, 1994; Mai, et al., 1996). The mouse total chromosome 13 paint was purchased from Cambio (CedarLane Laboratories Limited, Hornby, Ontario, Canada). The evaluation of metaphase spreads and interphase nuclei was performed using a Zeiss Axiophot microscope and a CCD camera (Photometrics/Optikon). Image analysis was performed using IPLab Spectrum H-SU2 (Signal Analytics, USA) and Gene Join (Yale University, USA) on a Power Macintosh 8100 computer. 100-150 interphases were evaluated in three independent experiments. Hybridization signals were measured with IPLab Spectrum/Multiprobe (Signal Analytics, USA), using the line measurement function. Relative fluorescent intensity per pixel (1 pixel=6.8 µm) was used to determine both single copy and amplified fluorescent signals. A signal is classified as amplified if the ratio between the relative fluorescent intensity per pixel of amplified vs. The relative fluorescent intensity per pixel of single copy signals is >2.

*Western blot analysis.* Peritoneal cavity cells were isolated one week LPS or pristane-treatment. Cells of control, LPS or pristane-treated mice were pooled for further analysis. 100 µg protein was loaded per lane and separated on a 10% SDS-PAGE gel (Mai, et al., 1994; Mai, 1994). Western blots were carried out using the ECL protocol as described (Mai, et al., 1994; Mai, 1994) and the 3C7 anti-c-Myc antibody (Evan, et al., 1985) as primary antibody at 200ng per blot. The secondary antibody was a peroxidase labeled anti-mouse antibody (Amersham) and was used at 1:20000 dilution. Densitometry was performed using the 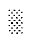 box function 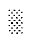 of the Sigma gel™ gel analysis program (Jandel, Scientific Software, USA).

*Southern blot analysis.* Peritoneal cavity cells were isolated one week after LPS or pristane-treatment. The cells of control, LPS or pristane-treated mice (6 mice per group) were pooled for further analysis. DNA was isolated as described (Mai, 1994) and 10 µg DNA was digested with EcoRI (Boehringer Mannheim, Canada) according to the manufacturer's protocol. Blotting, transfer and hybridization were carried out as outlined earlier (Mai, 1994). Equal loading and uniform transfer of the DNA were controlled by ethidium bromide staining. The filter was hybridized with a 1.2 kb PstI-fragment of the hamster DHFR gene (Chang, et al., 1978).

As shown recently, lymphoid and non-lymphoid cell lines amplify the DHFR gene as a result of c-Myc overexpression (Mai, et al, . 1996) . Moreover, p53-deficient mice show DHFR gene amplification and c-Myc upregulation within the same cells (Fukasawa, et al., 1997). However, in this latter system, it was not possible to directly assess whether c-Myc overexpression was the cause of DHFR gene amplification. To directly determine whether DHFR gene amplification occurred as a result of c-Myc overexpression in vivo, plasmacytoma (PCT)-susceptible Balb/c mice was examined that were injected i.p. with pristane (Potter, et al., 1992, Materials and Methods). Control Balb/c mice received i.p. injections of LPS (lipopolysaccharide) that elicites the transient activation of B cells concomitant with a transient upregulation of c-Myc protein levels, but does not lead to PCT genesis which requires the constitutive overexpression of c-Myc (Potter, et al., 1992).

Cells directly derived from the peritoneal cavity, the site of PCT diagnosis (Potter, et al., 1992), were analyzed for their c-Myc protein levels by quantitative fluorescent immunohistochemistry (Materials and Methods). Pristane elicited the elevation of c-Myc protein levels. When examined on a single cell level by quantitative fluorescence immunohistochemistry, the induction level of c-Myc protein reached 4- to 10-fold three days post pristane injection and remained elevated for the next four weeks. LPS induced a similar, but transient upregulation of c-Myc protein levels in B lineage cells. Non-treated peritoneal cavity cells exhibited low c-Myc protein levels. When the upregulation of c-Myc was analyzed in the total cell population of the peritoneal cavity by Western blots, the upregulation was visible, however less pronounced, with 2.6- and 1.4- fold induction for LPS vs. pristane-treatments, respectively.

Next examined is the genomic stability of the DHFR gene in the above groups of pristane-treated, LPS-treated and untreated Balb/c mice. Conventional Southern blot analysis did not show chromosomal amplification of the DHFR gene in the total genomic DNA of pristane- or LPS-treated cells isolated from the peritoneal cavity. Similar to previous findings (Mai, et al., 1996), Southern analysis suggested the partial rearrangement of the DHFR locus. This was only observed in pristane-treated mice. Since fluorescent *in situ* hybridization (FISH) is the most sensitive technique for the detection of genomic instability on a single cell level, being even more sensitive than the PCR (polymerase chain reaction)-based detection of chromosomal aberrations, and since it is therefore becoming the method of choice in clinical studies (Eckschlager, et al., 1996; White, et al., 1997; Afify, et al., 1997), this approach is used to evaluate genomic instability of the DHFR gene in peritoneal cavity cells. An increase in hybridization signals of the DHFR gene was observed after a single i.p. injection of pristane. LPS did not lead to an increase in fluorescent hybridization signals of the DHFR gene.

The relative fluorescent intensities of DHFR signals were measured using IPLab Spectrum software (Materials and Methods). The mean increase in DHFR signals in pristane-treated mice was 4.3-fold and affected 20-60% of all peritoneal cavity cells. The distribution of the signals suggested the presence of extrachromosomal elements carrying the DHFR gene.

Next, a test is run regarding whether the increase in DHFR hybridization signals was due to the increase in chromosome 13, the carrier of the mouse DHFR gene, or due to DHFR gene amplification. To distinguish between those two possibilities, a total chromosome 13-specific paint (Materials and Methods) is used and painted the peritoneal cavity interphase cells. In almost all interphases analyzed (>97%), chromosome 13 was present in two copies. Trisomy of chromosome 13 was observed din the remaining cells (<3%). The chromosome 13 paint also stained extrachromosomal elements that hybridized with DHFR confirming the data.

In the present report, pristane-treated Balb/c mice are shown to display an increase in c-Myc protein levels and DHFR hybridization signals as determined by FISH. In the majority of the analyzed cells (>97%), the latter can be attributed to DHFR gene amplification, with >97% of all cells exhibiting two copies of chromosome 13. <3% of all cells exhibited three copies of chromosome 13.

*The above in vivo findings allow two conclusions:* i) the DHFR gene is a molecular marker of c-Myc-dependent genomic instability following pristane-induction in plasmacytoma-susceptible mice *in vivo*, and ii) one may speculate that the amplification of the DHFR gene may be functionally important in c-Myc-induced genomic instability and neoplasia. While the first alternative is well documented in cell lines that overexpress c-Myc (Denis, et al., 1991; Mai, 1994; Mai,et al., 1996) and now also in the c-Myc overexpression-dependent mouse plasmacytoma, the second alternative remains hypothetical. As shown earlier, DHFR gene amplification and DHFR enzyme overexpression coincide (Luecke-Huhle, et al., 1996). One may therefore speculate that the amplification and overexpression of the DHFR enzyme, which is a key enzyme of folate metabolism, will lead to changes in the deoxynucleotide pool sizes, especially in the level of deoxythymidine triphosphate (dTTP). Changes in the nucleotide pool enhance mutation frequencies (Kunz, et al., 1994). Thus, DHFR overexpression may account in part for the accelerated acquisition of mutations and of further genomic instability. In addition, cellular proliferation and thus the statistically increased chance to generate a malignant clone may be enhanced due to DHFR overexpression. Interestingly, the potential to amplify the DHFR gene as well as the levels of DHFR gene amplification observed correlated with the metastatic potential in a rat tumor model (Luecke-Huhle, 1994).

In conclusion, the findings presented in this report confirm the previous in *vitro* data on c-Myc-dependent DHFR gene amplification in non-lymphoid and lymphoid cell lines of mouse, hamster, rat and human (Mai, 1994; Mai, et al., 1996) and show for the first time c-Myc-dependent DHFR gene amplification *in vivo.*

### Example 2

*Cell lines and tissue culture.* Human breast ductal adenocarcinoma T47D and mouse B lymphoma WEH1 231, were obtained from the American Type Culture Collection, Rockville, MD. Mouse plasmacytomas, MOPC 265 and MOPC 460D, the human colorectal carcinoma line, COLO320HSR, and primary human fibroblasts, GL30/92T, have been previously described (Jaffe et al., 1969; Mai et al., 1996; Mushinski, 1988). The spectrum of mouse B-lymphocytic cells lines has been presented in detail earlier (Mushinski et al., 1987). Cells were propagated in RPMI 1640 (Biofluids, Inc., Rockville, MD) supplemented with 10% heat-inactivated (30 minutes, 56°C) fetal bovine serum (Gibco/BRL, Germantown, MD), 2 mM glutamine, penicillin and streptomycin. Culture media for B-lymphoid cell lines also contained 5x10⁻⁵ M 2mercaptoethanol. In vitro line of mouse pre-B lymphocytes is generated by transformation of BALB/c bone marrow cells with A-MuLV (Rosenberg and Bltimore, 1976). These cells were subsequently transfected with pLXSN-bcl-2, a mouse bcl-2-expressing vector (Gurfinckel, et al. 1987), and pBabePuroMyc-ERTM, an expression vector (Littlewood et al., 1995) with which the human MYC protein can be activated by 100 nM 4-hydroxytamoxifen (4HT, Research Biochemicals International, Natick, MA). Also produced is a line of mouse fibroblasts in which MYC is upregulated by 4HT due to stable transfection of pBabePuroMyc-ERTM into y2 cells (Mann, et al., 1983).

*Cloning and sequencing of mouse cyclin D2 cDNA and* 5' *genomic flank.* A cDNA library of the mouse pre-B cell, 18-81, in lambda ZAP-2, was screened under relaxed conditions with Cyll (Matsushime et al., 1991), a partial cDNA for murine cyclin D1, from Dr. Charles Sherr. Several clones that encoded mouse cyclin D2 were isolated, rescued as pBlueScript clones, characterized and sequenced. A probe derived from the clone with the longest (1255 bp) insert was sequenced and found to have a coding region identical to the mouse cyclin D2 cDNAs in the literature (Kiyokawa et al., 1992). This probe was used to screen a partial EcoRI library of BALB/c liver DNA in EMBL-4 arms, from Drs. Linda Byrd and Konrad Huppi. One positive clone that contained a 17.1-kb insert was isolated, purified and digested to completion with EcoRI. Only one of the 3 EcoRI fragments that were generated from 2 internal EcoRI sites, a 5.4-kb fragment, hybridized with the 5' end of the cyclin D2 cDNA probe, and it was subcloned into pBlueScript for further study. Partial sequencing of this fragment revealed that the 3' 505 base pairs were identical to the 5' portion of our cyclin D2 cDNA and that of Kiyokawa et al. (1992). The 3' 194 base pairs contained the AUG and an open reading frame, and the adjacent 301 upstream base pairs contained the 5' untranslated sequence of the cDNA. The remainder was considered 5' flank in which regulatory motifs might be expected. The complete sequence of the mouse 5' flank is being generated and are reported elsewhere.

*Electrophoretic mobility shift and supershift assays.* Whole cell extracts were prepared as described (Mai and Jalava, 1994) from WEHI 231 mouse B-lymphoma cells and the mouse plasmacytomas MOPC 265 and MOPC 460D. All mobility shift reactions as well as supershifts were performed at room temperature. If not indicated differently, 5 mg of cellular protein were incubated for 5 minutes with 1 mg of non-specific competitor DNA (salmon sperm DNA) followed by a 30-minute incubation with 0.3 ng 32P-end-labeled oligonucleotides E1, E2, E3, and y (Figure 9), in low salt buffer (10 mM HEPES-NaOH, pH 7.9, 60 mM KCI, 1 mM EDTA, 1 mM DTT, 1 mM protease inhibitor AEBSF (Calbiochem, San Diego, CA), and 4% Ficoll 400. Gell electrophoresis was performed on 5% non-denaturing polyacrylamide gels in 22.5 mM Tris-borate/0.5 mM EDTA (Sambrook et al., 1986) at 8V for 4 hours. The gels were dried and subjected to autoradiography. Supershift analyses were carried out as follows: 5 mg of whole cell extracts were incubated with 1 mg non-specific competitor DNA (salmon sperm DNA) in low salt buffer (see above). Thereafter, antibodies were added at the concentrations indicated below for 30 minutes at room temperature prior to the addition of 32P-end-labeled oligonucleotides. Gel electrophoresis was performed as above. All antibodies were purchased from Santa Cruz Biotechnology Inc., Santa Cruz, CA, except the monoclonal anti-MAX antibody, which was obtained from Dr. Achim Wenzel, Deutsches Krebsforschungszentrum, Heidelberg, Germany and the anti-c-MYC polyclonal antibody and antiserum, which were kind gifts from Dr. U. Deutschel, Basel Institute for Immunology, Basel, Switzerland. Purified antibodies were used at 100 ng per reaction; the polyclonal anti-c-MYC antiserum and the respective pre-immune serum at 1 ml per reaction.

*Assays for genomic instability and gene amplification.* Gene dosage was examined using Southern blot analysis (Southern et al., 1975) and fluorescent *in situ* hybridization (FISH) of metaphase chromosomes (Mai et al., 1995). Evaluation of metaphase spreads and interphase nuclei was performed using a Zeiss Axiophot microscope and a CCD camera (Optikon/Photometrics). 100 -500 metaphases and interphases were evaluated in each of three independent experiments. Extrachromosomal fluorescent signals were considered specific when they also stained with 4', 6' diamidino-2-phenylindole (DAPI) (1 mg/ml) or propidium iodide (P1) (1 mg/ml) (Mai et al., 1996).

*RNA isolation and northern blotting.* Total RNA or Poly(A)+RNA was isolated from cells as previously reported (Mushinski, et al., 1987). 5 mg of Poly(A)+RNA or 15 mg of total RNA were fractionated on a 1% agarose gel containing formaldehyde. The RNA was transferred to a HybondN membrane (Amersham, Arlington Heights, IL) by capillary blotting and hybridized with 32P-labeled cDNA probes as indicated in the figure legends. Radioactive labeling was performed with the Nick Translation System (GIBCO/BRL, Germantown, MD) according to the manufacturer's protocol. The membranes were hybridized overnight with 3x10⁶ dpm/ml probe, washed with 0.1 x SSC, 0.1% SDS at 20°C and exposed to X-ray film overnight. For sequential hybridization of the same blot with different probes, membranes were stripped with boiling water.

*Probes.* A 700-bp Pstl-fragment of our mouse cyclin D2 cDNA was used to probe Southern and northern blots, and the genomic clone was used as a probe for FISH. A similar strategy was used to isolate cDNAs for mouse cyclins D1 and D3 (Hamel and Hanley-Hyde, 1997, generous gifts from Paul Hamel, University of Toronto). The human cyclin D2 cDNA was from Gordon Peters. It was used as a 1.2-kb Notl-Xhol-fragment. The ribonucleotide reductase R1 (RNR1) probe was a 1.5-kb BamHI fragment of mouse RNR1 cDNA (Thelander and Berg, 1986). The cDNA clone pMc-myc54 (Stanton et al., 1983) for mouse c-Myc was from Kenneth B. Marcu, from which a 0.6-kb Sst I-Hind III fragment was used as an exons2+3 probe for the Myc sequences expressed in pBabePuroMyc-ERTM (Littlewood et al., 1995). Mouse cyclins C and E probes were gifts of Steven Reed. The cDNA for the 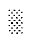 housekeeping gene 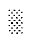 glyceraldehyde phosphate dehydrogenase (GAPDH) was from Dr. Marc Piechaczyk (Fort et al., 1993).

*Western blotting.* Western blots were performed on lysates of pre-B cell cultures as previously described (Mischak et al., 1993) except that protein concentration was determined using the BCA Protein Assay (Pierce), and 10 mg were loaded per lane. The immunoreactive bands were recognized by the ECL Western blotting detection system (Amersham, Arlington Heights, IL). The anti-cyclin D2 (M-20) was purchased from Santa Cruz Biotechnology (Santa Cruz, CA). The anti-actin (clone AC-40) was from Sigma ImmunoChemicals (St. Louis, MO). The HRP-Goat anti-Rabbit IgG and HRP-Goat anti-Mouse IgG and IgM were purchased from Axell (Westbury, NY) .

Northern blot analysis of cyclin D2 expression in murine B-lymphocytic lines with different degrees of B-cell maturation. Figure 8 shows a blot of poly(A)+ RNA from a series of mouse B-cell lymphoma cell lines of increasing maturation from left to right (Mushinski et al., 1987). When normalized to the GAPDH control hybridization signals, the highest level of expression of the predominant cyclin D2 mRNA (6.5-kb) was seen in the four plasmacytomas (lanes 11 - 14). In addition, several smaller cyclin D2 mRNAs are prominent, chiefly in these four lanes. These four lanes are the B cells with the highest Myc mRNA content. This blot was stripped and rehybridized with other murine cyclin D probes. The cyclin D3 probe revealed strong 2.3-kb bands in 4 cell lines of early B lymphocytes, but barely detectable levels in plasmacytomas. The cyclin D1 probe showed a very strong 3.8-kb band in the myeloid-pro-B line in lane 1, strong bands in 2 B-cell lines, lanes 5 and 8, but very low levels in the remaining RNA samples. Cyclin E transcripts were virtually undetectable. This pattern of high levels of Myc and cyclin D2 mRNA was also seen in Northern blots of RNA from 45 additional plasmacytomas that included tumors with t(12;15) and t(6;15) translocations and tumors without translocations but with Myc upregulation due to stable integration of Myc-expressing recombinant retroviruses (Mushinski, 1988).

The 5' flanking region of the cyclin D2 gene has several CACGTG-motifs that bind c-MYC/MAX. Partial sequencing of the 5' flank revealed four CACGTG motifs, putative 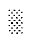 EMS (E-box MYC Sites) 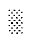 (E1-E4) at positions -867, -1487, -2988 and -3201 from the translation start site, as well as a variant motif, CAGGTG (y) at position -3475. The positions and adjacent sequence of three of the EMS motifs and y are indicated in Figure 9. A dot-matrix display shows bases that are identical in mouse cyclin D2 5' flank and the published 1624 bases upstream of the human AUG translation start site in the human cyclin D2 gene (Brooks et al., 1996), indicating a strong similarity between the mouse and human 5' flanks. Note that the 5' flank of the human cyclin D2 gene also contains at least two cacgtg motifs. These findings prompted analysis of three of the EMS motifs in the 5' flank of mouse cyclin D2 for their ability to bind MYC/MAX (Blackwood and Eisenman 1991), and to study whether the cyclin D2 locus would be amplified, overexpressed or both, under conditions of c-Myc overexpression.

Oligonucleotides containing four of the above-mentioned five E boxes were chosen for the mobility-shift analyses: oligonucleotides E1-E3 (EMS motifs 1-3) and y (the variant motif). Purified MYC and MAX bound to E1-E3 but not y. In 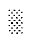 super-shift 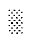 experiments, the DNA-protein complex I was found to contain both MYC and MAX in logarithmically growing plasmacytoma MOPC 265 cells (Figure 10). Under these conditions, antibodies directed against c-Fos, Mad, Mxi-1 or USF, an unrelated E-box-binding protein, did not disrupt or supershift complex I, whereas antibodies directed against MYC and MAX did. These data suggested that, under proliferative conditions, MYC and MAX were present in plasmacytoma cells, bound to canonical EMS motifs, and appeared as complex I in mobility shifts and supershifts.

Mouse and human tumors with c-Myc overexpression have Southern blot evidence of amplification of the cyclin D2 locus. The stability of the cyclin D2 locus was characterized in two mouse B-lymphoid lines, MOPC 460D, a plasmacytoma that constitutively overexpresses c-Myc due to Myc/lg chromosome translocation (Mushinski, 1988), and WEH1 231, a lymphoblastoid tumor with low MYC protein levels (Mai et al., 1996). Southern blot analyses showed that the cyclin D2 gene was amplified in MOPC 460D (Figure 11, panel A, filled arrowheads), but not in WEHI 231 cells. As in our previous studies, mouse ribonucleotide reductase R1 (RNR1), a gene that is retained as single copy gene irrespective of MYC protein levels, was used as a reference gene (Figure 11, panel B).

These analyses were extended to human cell lines: the colon carinoma line COLO320HSR, a classic example of c-Myc gene amplification and overexpression (28-fold higher MYC protein levels than GL30/92T primary human fibroblasts); and the breast cancer line T47D, which expresses 11 times higher c-MYC protein levels than GL30/92T (Mai et al., 1996). As in our previous studies, human cyclin C, a gene that is retained as single copy gene irrespective of MYC protein levels, was used as a reference gene. The cyclin C gene was not amplified in any of the human cell lines (Figure 11, panel B). COLO320HSR and T47D displayed amplified bands of cyclin D2 gene hybridization (Figure 11, panel A, filled arrowheads), while primary human fibroblasts did not. In T47D, the gene for cyclin D2 is partially deleted as indicated by missing genomic bands in the Southern blot (see open arrowheads in Figure 11). Such deletions may reflect an additional form of genomic instability of this locus in cells that overexpress Myc.

Cyclin D2 amplification involves the generation of extrachromosomal elements in COLO320HSR and MOPC 460D. DAPI or PI staining of metaphase chromosome spreads of COLO320HSR, a human adenocarcinoma line, showed the presence of extrachromosomal elements (ECEs). Fluorescent *in situ* hybridization (FISH) showed amplified signals of the cyclin D2 gene on chromosomes and the ECEs (Figure 12). A similar analysis of the BALB/c plasmactoma MOPC 460D also showed ECEs that contained cyclin D2 sequences (Figure 12). In agreement with the Southern data (Figure 11), FISH hybridization of COLO320HSR and MOPC 460D showed neither evidence of amplification of the RNR1 gene nor extrachromosomal elements that hybridized with an RNR1 probe.

Induced upregulation of MYC activity in mouse pre-B cells results in cyclin D2 amplification and increased mRNA and protein after three days. A mouse pre-B cell line derived from bone marrow cells by transformation with Abelsonmurine leukemia virus (A-MuLV) was stably transfected with pBabePuroMyc-ERTM, an inducible MYC expression vector that is activated by 4HT. After three days of stimulation by 4HT, numerous ECEs may be staining of chromatin and DNA. Several of the ECEs were shown to contain extra copies of the cyclin D2 gene by FISH analysis (Figure 12). This evidence of genomic instability and of cyclin D2 gene amplification was not seen in the same cells without prolonged tamoxifen stimulation (Figure 12).

Northern blots were prepared from total RNA from the A-MuLV-transformed pre-B cells before and after stable introduction of pBabePuroMyc-ERTM, and after different periods of stimulation by 4HT. Figure 13 shows the results of successive hybridizations of this blot with cyclin D2 and other germane probes. Cyclin D2 message expression is clearly elevated after 4 and 6 days of MYC activation by 4HT when compared to the ethidium bromide-stained 28S ribosomal RNA bands in each lane. Transcripts from the retroviral pBabePuroMyc-ERTM in the stably transfected line, shown in the right panels, are detected with a Myc exon 2+3 probe. Endogenous 2.4-kb c-Myc expression was repressed after activation of the exogenous MYC-ER by 4HT, as shown by hybridization with Myc exon 1 probe, since exon 1 is not present in the MYC-ER-containing expression vector. There was no detectable mRNA for cyclin D1, and no significant change in mRNA level of cyclin D3 was seen during the treatment with 4HT.

Western blots of lysates from the 4HT-treated cells described in the preceding paragraph were probed with anti-cyclin D2 antibody and, as a loading control, with anti-actin antibody. As shown in Figure 13, cyclin D2 protein levels gradually rose in parallel with the levels of cyclin D2 mRNA after MYC activation by 4HT in the pBabePuroMyc-ERTM-containing cells but not in the pre-B cells that lack this vector.

Inducing upregulation of MYC activity in mouse fibroblasts also leads to cyclin D2 amplification and increased cyclin D2 mRNA. A mouse fibroblast line, derived by transfection of y2 cells with pBabePuroMyc-ERTM, was stimulated with 4HT to induce increased MYC activity. After three days of stimulation by 4HT, numerous cyclin D2-containing ECEs are seen in metaphase chromosome spreads and in interphase nuclei (Figure 12). This evidence of genomic instability and of cyclin D2 gene amplification were not seen in the same cells without prolonged tamoxifen stimulation (Figure 12). A control FISH study of 4HT-stimulated y2 cells that do not bear the MYC-ERTM expression vector showed no cyclin D2-hybridizing ECEs (Figure 12). Northern blots were prepared from total RNA from the fibroblasts with and without stable integration of pBabePuroMyc-ERTM, and after different periods of stimulation by 4HT. Figure 13 shows the results of successive hybridization of this blot with cyclin D2 and other probes. As with the B cells, cyclin D2 message expression is clearly elevated after several days of MYC activation by 4HT, when compared to the GAPDH loading control.

The data presented in this report indicate that the mouse cyclin D2 gene has at least four MYC-MAX-binding EMS motifs 5' of its first exon. Oligonucleotides that contain any of these motifs bind purified MYC and MAX proteins, as well as MYC and MAX proteins that are present in lysates of mouse plasmacytoma cells. The presence of at least two CACGTG motifs upstream of the human cyclin D2 coding region suggests that these motifs have been evolutionarily conserved due to some essential role in normal cell physiology. This region of high structural homology has been recently suggested to play a role in regulation of cyclin D2 expression, but the role of MYC in genomic instability or cyclin D2 expression was not addressed directly (Jun et al., 1997). The details of how MYC/MAX binding to each individual EMS motif affects cyclin D2 transcription are the subject of another report (J. Hanley-Hyde, in preparation).

A direct role for MYC in cyclin D2 gene amplification was first suspected when a coupling was observed between Myc overexpression and amplification of the cyclin D2 gene in established tumors. Amplification of cyclin D2 was first seen in Southern blots of two human cell lines, COLO320HSR and T47D, which were known to have c-Myc amplification and overexpression. Similar evidence of cyclin D2 amplification was also found in mouse plasmacytomas that did not have c-Myc gene amplification but which did have c-Myc overexpression, due to chromosomal translocations. Extracts from these cells were shown to contain MYC/MAX complexes that bound in vitro to three of the CACGTG-containing oligonucleotides that is identified on the 5' of cyclin D2.

The cyclin D2 amplification that was detected in mouse plasmacytomas is associated with enhanced mRNA levels on RNA blots; more transcripts are found in plasmacytomas than in other cell lines that do not have c-Myc-activating chromosome translocations. Such increased expression of other members of the G1 cyclins, D1, D3 and E was not found in plasmacytomas, indicating that this was a special attribute of cyclin D2. Since cyclin D1 is not thought to be expressed in normal B lymphocytes (Sinclair et al., 1994), it may be noteworthy that two mouse B-cell lines exhibit substantial cyclin D1 expression: BALB 1437 and BAL 17. The mechanisms responsible for the overexpression of cyclin D1 in these lines have not been investigated.

To directly implicate MYC levels in the induction of cyclin D2 amplification, the effects of inducible overexpression of Myc in mouse pre-B cells is studied using a tamoxifen-activated pBabePuroMyc-ERTM chimeric expression vector. Since amplification of genes occurs gradually, over successive replication cycles. Instead, concentration is on the state of the locus and its expression over several days of 4HT stimulation. 4HT had no effect on the cyclin D2 of parent A-MuLV-transformed pre-B cells. *In situ* hybridization showed no evidence of genomic instability, and mRNA expression remained very low. In the cells with activated MYC-ERTM chimera, extrachromosomal elements, also called double-minutes or polydispersed circular DNA, episomes and extrachromosomal DNA (Cohen et al., 1997), that hybridized with the cyclin D2 probe, appeared after three to four days, indicating increased genomic instability. At these same time points, blots of RNA and cell lysates isolated from these cells began to show increased expression of cyclin D2 mRNA and protein. The data obtained to date do not require upregulation of either RNA transcription or changes in RNA stability. Simple status-quo rates of expression would yield increased steady-state levels of mRNA and protein if the template were increased, such as by the amplification that is demonstrated. Such a mechanism is also be responsible for the high levels of cyclin D2 mRNA in plasmacytomas, secondary to their constitutive expression of high levels of c-Myc mRNA and protein. It is interesting to note that Southern blots of DNA from pre-B cells after 3 days of 4HT-induction did not show increased cyclin D2 hybridization signals like those that were seen in well-established tumor cells that have experienced high MYC levels for many generations. This may be connected with the nature of DNA in extrachromosomal elements that have been amplified for a short time. This is not surprising, since it has been well documented in the literature that FISH analysis is a much more sensitive technique for identification of amplification of genes than Southern blotting (Cohen et al., 1997).

Such a connection between MYC expression and cyclin D2 amplification is probably not limited to B lymphocytic tumors, because amplified cyclin D2 in human colorectal and breast carcinomas is seen. In addition, a gradual increase in cyclin D2 expression in mouse fibroblasts is found when MYC is overexpressed and activated by 4HT treatment of cells that bear the pBabePuroMyc-ERTM expression vector.

This MYC-associated genomic instability may have another possible consequence: the frequent aneuploidy seen in plasmacytomas (and other cancer cells) that express high MYC levels and have been passaged for extended periods of time *in* vivo or *in vitro.* It has been reported that tumor-specific initiating non-random chromosome translocations become increasingly difficult to recognize with repeated passages due to accumulations of additional, presumably random, chromosomal aberrations (Coleman et al., 1997). However, it is important to emphasize that this MYC-associated tendency to amplification is locus-specific. It has been demonstrated previously for Dhfr and in this paper for cyclin D2, but is has been determined that high Myc expression produces no such amplification in the genes encoding ornithine decarboxylase, syndecan-2, glyceraldehyde-3-phosphate-dehydrogenase and cyclin C (Mai et al., 1996).

It possible to construct a hypothetical model for how Myc overexpression and the genes that are amplified in its presence might work together toward neoplasia. It is their common role in promoting cell cycle progression and cell proliferation that produces a potent combination favoring induction, promotion or progression of neoplastic transformation. Overexpression of Myc has been shown to shorten the G1 phase of the cell division cycle (Karn et al., 1989), which favors further mutations by curtailing the period available for cells to assess and repair DNA damage before it is duplicated in S phase. A similar effect would be expected from overexpression of cyclin D2, an important G1 cyclin. High levels of such cyclins are also foreshorten G1 and rush cells prematurely into S by titrating out cdk inhibitors such as p21 and p27. Perhaps such changes are responsible for the transformed characteristics that are induced by overexpression of cyclin D1 in fibroblasts (Jiang et al., 1993). Cyclin D1 amplification and overexpression is a well-known step in various cancers (Motokura and Arnold 1993; Wang et al., 1994; Zho et al., 1995). Amplification and/or overexpression of cyclin D2 may have similar effects. Overexpression of cyclin D2, along with D1 and D3, has been found in mouse skin neoplasms and has been associated with tumor progression (Zhang et al., 1997). Similar to our finding of cyclin D2 gene amplification in COLO320HSR, Leah et al. (1993) reported the amplification of this cyclin in a subgroup of colorectal carcinomas. What is more, inappropriate expression of cyclin D2 also occurs as a result of retroviral integration in retrovirus-induced rodent T-cell lymphomas (Hanna et al., 1993). Finally, the expression of G1 cyclins and their control of the cell division cycle is known to vary between normal and transformed cells (Hamel and Hanley-Hyde 1997).

As mentioned above, amplification associated with Myc overexpression is not random, but is locus-specific. Another gene that is amplified by Myc overexpression, Dhfr, is a key enzyme of folate metabolism, and it is essential for DNA synthesis. High levels of the product of this gene may contribute of this gene may contribute to maintenance of cell proliferation. High copy number of Dhfr genes, e.g., following amplification, have been correlated with the metastatic potential of tumor cells in a rat carcinoma model (Lueke-Huhle, 1994). Similarly, the gene encoding ribonucleotide reductase R2 subunit, which is required for dNTP (deoxynucleoside triphosphate) synthesis, is amplified as a result of c-Myc deregulation (T.I. Kuschak et al., submitted) Furthermore, it has been reported recently that the R2 protein is a malignancy determinant in neoplastic cells (Fan et al., 1996).

A model in which MYC promotes genomic instability fits the data reported here and offers a potential explanation for the frequent observation that high expression of c-Myc contributes to neoplastic development. Not only does it force cells through the G1 phase of the cell cycle abnormally rapidly, but also, if they escape apoptosis, these cells may suffer increased genomic instability in certain loci, which compounds the precocious cell cycling problem. This makes it possible, and indeed likely, that such cells will accumulate additional genomic alterations and complete the multi-step process of neoplastic transformation.

### Example 3

To determine whether the locus-specific amplification of the DHFR gene occurred as a result of c-Myc overexpression *in vivo*, an animal model of c-Myc-dependent neoplasia is examined, the mouse plasmacytoma (Potter, et al., 1992). Using plasmacytoma-susceptible Balb/c mice, it is analyzed whether DHFR gene amplification occurred during pristane-induced plasmacytomagenesis. Balb/c mice are examined that were injected i.p. with pristane (Potter, et al., 1992). Control Balb/c mice received i.p. injections of LPS (lipopolysaccharide) that elicites the transient activation of B cells concomitant with a transient upregulation of c-Myc protein levels, but does not lead to PCT genesis which requires the constitutive overexpression of c-Myc (Potter, et al., 1992).

c-Myc protein levels of cells directly isolated from the peritoneal cavity (Potter, et al., 1992) were analyzed by quantitative fluorescent immunohistochemistry. Pristane elicited the elevation of c-Myc protein levels (Table 4). The induction level reached 4 to 10 fold three day post pristane injection and prevailed elevated for the next four weeks. LPS induced a similar, but transient upregulation of c-Myc protein levels in B lineage cells (Table 4). Non-treated peritoneal cavity cells exhibited low c-Myc protein levels (Table 4).

Next the Balb/c mice are examined to determine whether DHFR gene amplification in Balb/c mice was induced as a result of the above treatments. Peritoneal cavity cells were analyzed by fluorescent *in situ* hybridization (FISH). The amplification of the DHFR gene was observed after a single i.p. injection of pristane (Table 4). Interestingly, LPS did not lead to the amplification of the DHFR gene (Table 4). These findings allow one to conclude that the DHFR gene is a molecular marker of c-Myc-dependent genomic instability *in vivo.* Moreover, these findings confirm our previous in vitro data on c-Myc-dependent DHFR gene amplification in non-lymphoid and lymphoid cell lines of mouse, hamster, rat and human. Interesting, the fully developed plasmacytoma also exhibited DHFR gene amplification.

As shown earlier, DHFR gene amplification and DHFR enzyme overexpression coincide (Luecke-Huhle, et al., 1996). It is not known whether the amplification of the DHFR gene is functionally important in c-Myc-induced genomic instability and neoplasia. Based on the fact that DHFR gene amplification occurs both early and late during pristane-induced plasmacytomagenesis in Balb/c mice, and this amplification event plays a role during the initiation of genomic instability and neoplastic transformation (Figure 15). It has been described by others that imbalances in the nucleotide pool enhance mutation frequencies (Kunz, et al., 1994). Consistent with these findings, the amplification and overexpression of DHFR enzyme, which is a key enzyme of the folate metabolism, may lead to changes in the nucleotide pool, especially in the dTTP pool, which affect both DNA synthesis and mutation frequencies. Thus DHFR amplification and overexpression may account in part for accelerated acquisition rates of mutations and for further genomic instability. Moreover, DNA synthesis and cellular proliferation and thus the statistically elevated probability to generate a malignant clone may be enhanced due to DHFR amplification and overexpression. It is noteworthy that the potential to amplify the DHFR gene as well as the levels of DHFR gene amplification correlate with the metastatic potential in a rat tumor model (Luecke-Huhle,, 1994).

### Example 4

Here, genomic instability *in* vivo is studied in different organs of p53^{-/-} mice (4-6 weeks old), with age-matched p53 homozygous (p53^{+/+}) mice as controls. In all p53^{-/-} tissues examined, a substantial percentage of cells contained abnormal numbers of centrosomes and displayed aneuploidy. Moreover, c-Myc overexpression was observed in 5-15% of p53^{-/-} cells. In these cells, dihydrofolate reductase (DHFR), carbamoyl-phosphate synthetase-aspartate transcarbamoyl-dihydroorotase (CAD) and c-myc genes exhibited gene amplification. Apoptosis of cells, which displayed abnormal numbers of centrosomes, aneuploidy, gene amplification and c-Myc overexpression, was frequently observed.

*Aneupolidy in p53*^{*-/-*} *mice*. Genomic instability is examined in different organs of clinically healthy p53^{-/-} mice (4-6 weeks old) by cytogenetically assessing chromosome ploidy. Age-matched parental p53^{+/+} mice were used as controls and then characterized as spleen-, thymus, and bone marrow-derived cells, as well as skin- and spleen-derived fibroblasts. These analyses revealed aneuploidy; hyperdiploid, hypo-, hypertetraploid, and polyploid metaphase plates were present in all organs examined (Figure 16). The percentage of aneuploid mitotic plates varied between individual mice; the mean frequencies were 25.6% in the thymus, 34.8% in fibroblasts, 10% in the bone marrow, and 20% in the spleen (Figure 16). No aneuploidy was observed in p53^{+/+} mice (Figure 16).

Cytogenetic studies also revealed that five percent of all metaphases present in the p53^{-/-} fetal liver hematopoietic cells were aneuploid (Figure 16). In contrast, there was no evidence of aneuploidy in age-matched p53^{+/+} fetal liver hematopoietic cells.

*Abnormal centrosome amplification in vivo in p53*^{*-/-*} *mice.* p53 has been implicated in the regulation of centrosome duplication, and multiple centrosomes are generated in p53^{-/-} embryonic fibroblasts (MEFs) during a single cell cycle (Fukasawa et al., 1996). In the present work, the chromosome instability is tested and observed in p53^{-/-} mice was associated with multiple centrosomes per cell *in vivo.* Spleen-, thymus-, and bone marrow-derived cells were immunostained with anti-γ-tubulin antibody to identify centrosomes (reviewed in Oakley, 1992; Joshi, 1994), and the number of centrosomes per cell was scored. The numerical distribution of centrosomes in various organs of p53^{+/+} and p53^{-/-} mice is summarized in Figure 17. More than 99% of the interphase p53^{+/+} cells contained one or two centrosomes, most likely depending on their duplication cycle, while 20-30% of the interphase p53^{-/-} cells contained >2 centrosomes. A typical example of normal vs. Aberrant centrosome duplication is shown in Figure 17. Thus, as previously observed *in vitro* in p53^{-/-} MEFs, *in vivo* that mitotic p53^{-/-} cells frequently displayed abberant spindles, organized by multiple copies of centrosomes. However, as reported (Fukasawa et al., 1996) in some mitotic p53^{-/-} cells, abnormally amplified centrosomes sequestered to the poles to form bipolarity. These results imply that abnormal amplification of centrosomes occurs *in vivo,* and this may lead to chromosome instability in p53^{-/-} mice.

*Gene amplification in p53*^{*-/-*} *mice.* To evaluate the genomic (in)stability of single genes in p53^{-/-} mice *in vivo,* fluorescent *in situ* hybridization (FISH) was performed. Then analysis is conducted on the c-myc gene, which is frequently translocated and/or amplified in tumors (for review, see Marcu et al., 1992; Bishop, 1995), and the DHFR gene, whose genomic instability is altered following either drug selection (Stark, 1993), growth factor (Huang and Wright, 1994) or c-Myc overexpression (Denis et al., 1991; Mai, 1994; Mai et al., 1996). The CAD and the ribonucleotide reductase R1 (R1) genes were also examined. The CAD gene encodes a trifunctional enzyme of the pyrimidine biosynthesis and has been shown to be amplified after exposure to PALA (N-(phosphonoacetyl)-L-aspartate) (Otto et al., 1989; Yin et al., 1992; Livingstone et al., 1992). R1 forms a functional ribonucleotide reductase molecule in association with a second subunit, ribonucleotide reductase R2 (R2). The genomic stability of R1 is maintained even in malignant cells (Mai et al., 1996) and served as a control.

In all p53^{-/-} tissues examined, there was detected an increase in fluorescent signals as observed by FISH for DHFR and c-myc as well as for CAD in both interphases and metaphases (Figure 18 and Figure 19). In contrast, no increase in fluorescent signals for the above genes was detected in age-matched control p53^{+/+} mice (Figure 18). R1 was present as single copy gene in both p53^{-/-} and p53^{+/+} cells.

An increase of fluorescent signals as detected by FISH may be due to two forms of genomic instability, karyotypic instability (gain of chromosomes) and/or gene amplification. The latter may also involve the formation of extrachromosomal elements which is frequently found in early stages of tumorigenesis (Wahl, 1989). Extrachromosomal elements were observed hybridizing with either DHFR or c-myc in all organs of 4-6 week old p53^{-/-} mice; a representative picture is shown in Figure 18. *c-Myc overexpression and amplification of c-myc, DHFR and*

*CAD in the same p53*^{*-/-*} *cells.* Since c-Myc overexpression has been shown to be associated with locus specific gene amplification, the levels of c-Myc protein in p53^{-/-} bone marrow-, thymus-, spleen-derived cells as well as fibroblasts were examined by quantitative fluorescent immunohistochemistry (Materials and methods) . Five to fifteen percent of the p53^{-/-} cells in all tissues examined showed a two- to eightfold increase in c-Myc expression, while less than 1% of p53^{+/+} cells expressed detectable levels of c-Myc protein. To determine whether c-Myc deregulation and genomic instability of single genes occurred within the same p53^{-/-} cell(s) *in vivo,* the Combined Protein/FISH Analysis (CPFA) (Materials and methods) was developed. This assay allows the simultaneous analysis of c-Myc protein levels and FISH hybridization signals in the identical cell(s) *in vivo.* It therefore allows one to conclude whether c-Myc overexpression is associated with genomic instability in the same cell(s) *in vivo.* A similar technique has been independently established by Hessel et al. (1996).

Using CPFA, c-Myc overexpression and amplification of DHFR, c-myc, and CAD genes was observed, but not of the R1 gene within the same individual p53^{-/-} bone marrow, thymus, spleen-derived cells as in primary fibroblasts (Figure 19, panels a-a 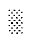 , b-b 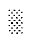 ). Thus, locus-specific gene amplification in p53^{-/-} mice appears to be accompanied by c-Myc overexpression.

*Apoptosis of genomically altered cells in p53*^{*-/-*} *mice.* Despite the extensive genomic alterations present in fetal and neonatal life, p53^{-/-} mice develop without apparent abnormalities (Donehower et al., 1992; Jacks et al., 1994). One possibility is that cells with deleterious genomic alterations may be efficiently eliminated by apoptosis. Therefore it was determined that apoptosis occurred in organs and fibroblasts from p53^{-/-} mice. Apoptosis frequently occurred in tissues and fibroblasts of p53^{-/-} mice, with cells displaying chromatic condensation characteristic of apoptosis and a positive TUNEL reaction (Figure 20). Cytogenetic and FISH analyses of p53^{-/-} cells revealed the frequent presence of morphologically atypical chromosomes (Figure 20) in contrast to aneuploid plates with the typical chromosome morphology (Figure 20). Moreover, morphologically atypical chromosomes exhibited a strong positive TUNEL reaction in all organs examined (Figure 20). Such morphologically atypical chromosomes were exclusively observed in p53^{-/-} mice. They seem to undergo chromatid fragmentation and degradation and often showed gene amplification as well (Figure 20). Apoptotic cells characteristically displayed multiple copies of centrosomes (Figure 20), aneuploidy, c-Myc overexpression (Figure 20), and/or gene amplifications (Figure 20).

p53^{-/-} mice develop a variety of tumors early in life (Donehower et al., 1992; Jacks et al., 1994), but the mechanisms underlying this tumor predisposition have remained elusive. Here, it is shown that cells directly isolated from p53^{-/-} mice display extensive aneuploidy and gene amplification. This enhanced genomic instability is coupled with the high susceptibility and frequency of tumor development in these mice.

Genomic instability initiates during embryonic development and increases during life; haematopoietic cells of the fetal liver displayed 5% of aneuploidy, while all organs of young (4-6 week old) exhibited higher levels of aneuploidy (Figure 16).

Abnormal amplification of centrosomes occurs in p53^{-/-} mice *in vivo*, in all cell types tested. The adverse effects of abnormal centrosome amplification are readily observed in cells undergoing mitosis. For instance, the formation of aberrant mitotic spindles organized by multiple copies of centrosome was frequently observed in p53^{-/-} mice. Such events are likely to impair chromosomal segregation, and induce karyotypic instability and aneuploidy.

Consistent with *in vitro* studies demonstrating that c-Myc expression is negatively regulated by p53 (Ragimov et al., 1993), it is found that in the absence of p53, all tissues became permissive to c-Myc overexpression. However, only 5-15% of p53 cells expressed high levels of c-My protein, suggesting that the additional event(s) may be required for c-Myc overexpression to occur and/or that cells overexpressing c-Myc undergo apoptosis (Figure 20; Evan et al., 1992; Packham and Cleveland, 1995).

The absence of p53 and the deregulation of c-Myc expression seem to cooperate during tumor development. For instance, c-Myc overexpression and lack of p53 have synergistic effects during lymphomagenesis in Eµmyc/p53^{+/+} and CD2-myc/p53^{-/-} mice (Blyth et al., 1995; Hsu et al., 1995). Thus, c-Myc may play an important role in the overall tumor susceptibility of p53^{-/-} mice by contributing to genomic instability, such as locus-specific gene amplification as well as increased proliferation rates (Karn et al., 1989).

It has been previously shown that cell lines overexpressing c-Myc display DHFR amplification, independent of species and tissue origins (Mai et al., 1996). Moreover, the c-myc gene was both translocated and amplified, and the protein was overexpressed in mouse plasmacytoma cells (Mai et al., 1995). In this study, the genomic stability of c-myc, DHFR, and CAD were tested, all of which were amplified concomitant with c-Myc overexpression in cells isolated from p53^{-/-} mice.

While no mutagens were used in this study, several groups have shown earlier that the administration of the drug PALA (N-(phosphonoacetyl)-L-aspartate) leads to the selection of drug-resistant cells with amplified CAD genes (Otto et al., 1989; Yin et al., 1992; Livingstone et al. 1992), and this occurs with an enhanced frequency in p53^{-/-} cells (Yin et al., 1992; Livingstone et al., 1992) A role for c-Myc in PALA-induced CAD amplification has not been described. Recent work suggests that c-Myc is involved in the transcriptional regulation of the CAD gene (Boyd and Farnham, 1997). Since c-Myc acts both as a transcription and replication factor, one may propose a role for c-Myc not only in the transcriptional activation, but also in the replication/amplification of the CAD gene. Consistent with this idea, it has recently been observed the PALA-dependent upregulation of c-Myc protein levels in p53^{-/-} fibroblasts. Moreover, PALA-induced c-Myc overexpression occurred prior to CAD gene amplification (SM). C-Myc overexpression may thus precede the genomic instability of the CAD gene as it does for DHFR (Mai and Jalava, 1994; Mai et al., 1996), cyclin D2 (Mai et al., 1997) and ribonucleotide reductase R2 (Kuschak et al., 1997). Further investigation shows that c-Myc deregulation is a necessary and limiting molecular event in CAD gene amplification.

Genomic instability in p53^{-/-} embryos and young mice affects several genetic loci, includes c-Myc overexpression, abnormal centrosome numbers and aneuploidy. Further genomic and molecular alterations, such as changes in the expression and/or half life of additional oncogenes, cell cycle related genes, growth factor-mediated signaling, DNA repair, etc., are conceivable, but have not been examined here. During the multistage process of carcinogenesis, all of the above events may ultimately contribute to the selection and evolution of neoplastic cell(s).

Despite extensive genomic alterations, p53^{-/-} mice mature without discernible abnormalities until tumors start to appear (Donehower et al., 1992; Jacks et al., 1994). As is show here, there is a high incidence of apoptosis in all p53^{-/-} tissues. In addition to interphase cells exhibiting chromosome condensation and a positive TUNEL staining, there is detected chromosomes with atypical morphology. These chromosomes stained in the modified TUNEL reaction and thus shows chromatid fragmentation. It is noteworthy that these apoptotic cells usually contained abnormally amplified centrosomes, expressed high levels of c-Myc protein, and displayed aneuploidy and gene amplifications. Thus, many of the genetically abberant cells may be eliminated through p53-independent apoptosis. This may explain how p53^{-/-} mice seemingly develop normally. However, the yield of p53^{-/-} offspring from heterozygous crosses has been reported to be only ^{~}60% of the expected yield (Jacks et al., 1994), suggesting that some homozygous lethality occurs during embryogenesis. Indeed, genomic alterations were observed in a fraction of embryonic cells (Fukasawa et al., 1996, and this study). Since c-Myc has been implicated in apoptosis (reviewed in, Amati and Land, 1994; Harrington et al., 1994; Packham and Cleveland, 1995) and apoptosis occurs in the presence of elevated c-Myc expression in p53^{-/-} mice.

*Mice, cell suspensions, cell culture.* Mice were obtained from Taconic Farms (Ca, USA), with the exception of two p53^{+/+} parental C57B1/6 mice that were obtained from Charles River (Quebec, Canada). Nine p53^{-/-} and six p53^{+/+} mice between 4 and 6 weeks old were used to prepare single cell suspensions of spleen, bone marrow, and thymus. Ten-day-old skin- and spleen-derived primary fibroblasts (passage 0) were also obtained from these mice by explanting subcutaneous skin tissue pieces and spleen fragments into RPMI 1640 medium supplemented with 10% fetal calf serum, 2mM L-glutamin, and 1 mM sodiumpyruvate. Hematopoietic cells were prepared from fetal livers of 16-day-old embryos. Ten fetal livers were pooled for further analyses.

*γ-tubulin immunostaining.* Cells isolated from mice were seeded onto the slides. Samples were fixed in 3.7% formaldehyde in phosphate buffered saline (PBS) for 20 min at room temperature (RT). Cells were then incubated in the blocking solution (10% normal goat serum in PBS) for 1 h at RT. The samples were then incubated with anti-γ-tubulin antibody raised against CSREIVQQLIDEYHAATRPDYISWGTQ for 1 h at 37°C. The samples were then washed extensively in PBS, followed by incubation with fluorescein isothiocyanate (FITC)-conjugated goat anti-rabbit immunoglobulin G (IgG) for 30 min at RT. The samples were then washed extensively in Tris buffered saline (TBS), followed by 4',6' diamidino-2-phenylindole (DAPI) DNA staining (1 µg/ml). For each cell type, >400 cells were examined.

*c-Myc determination.* Quantitative fluorescent immunohistochemistry was used to determine c-Myc protein levels in single cells directly isolated from the mice. Following immunohistochemistry with 20 ng/slide of a monoclonal anti c-Myc antibody 3C7 (Evan et al., 1985) and a secondary antibody (anti-mouse IgG-Texas Red; Southern Biotechnology Associates, Inc., USA) at 10 ng/slide, images were acquired using a Zeiss Axiophot microscope, coupled to a CDD camera (Optikon/Photometrics), and the relative fluorescence intensity per pixel (one pixel = 6.8 µm) was analyzed on a Power Mac 8100, using IPLabSpectrum and Multiprobe software, version 3.1 (Signal Analytics, USA). One hundred to three hundred cells were evaluated per sample.

*Cytogenetics and fluorescent in situ hybridization (FISH).* Metaphase spreads for cytogenetic analysis, FISH and a modified TUNEL assay (see below) were performed according to standard protocols (Mai, 1994; Mai et al., 1996) using cells directly isolated from the mice. Analyses of interphase cells by FISH, CPFA (see below) and the TUNEL assay (Li et al., 1995) were carried out on cytospin preparations. FISH determination of metaphase chromosomes and interphase cells was performed as described (Mai, 1994; Mai et al., 1996). All probes used have been described elsewhere (Mai et al., 1996), except the CAD probe (a generous gift from Dr. George Stark, The Cleveland Clinic Foundation, OH). The number of metaphase plates evaluated for cytogenetic analyses was 50 per organ and 100 per fetal liver cells. The number of metaphase plates and interphases evaluated for FISH analyses was 100 per sample. Using the IPLabSpectrum and Multiprobe softwares (Signal Analytics, USA), amplified signals were determined with the 'Line Measurement' function; relative fluorescent intensities per pixel (one pixel = 6.8 µm) were measured for single and amplified hybridization signals. As signal is classified as amplified if the ratio between the relative fluorescent intensity per pixel of amplified vs. the relative fluorescent intensity per pixel of single copy signals is >2 in one hundred interphase cells.

*Combined protein/FISH analysis (CPFA).* To visualize c-Myc protein expression and genomic (in)stability within the same cells, immunohistochemical analysis and FISH are combined. Cells immobilized on slides were fixed (3.7% formaldehyde), permeabilized (0.2% Triton X-100), incubated with 20 ng/slide of the anti-c-Myc monoclonal antibody (3C7, Evan et al., 1985), followed by the secondary Texas Red-conjugated goat anti-mouse IgG (Southern Biotechnology Associates, Inc., USA) (10 ng/slide). The nuclei were counterstained with DAPI (1 µg/ml in PBS), photographed, and the positions were recorded. Thereafter, the slides were processed for FISH analysis as described (Mai, 1994; Mai et al., 1996). Cells in the recorded positions were re-evaluated for their respective gene copy numbers. A microscopic field of 10⁴ to 10⁵ cells was analysed, and 100 cells were evaluated per sample. As above, relative fluorescent intensity per pixel was determined using the IPLabSpectrum software (Signal Analytics, USA).

*Apoptosis assays.* The TUNEL assay was performed on interphase cells as described (Li et al., 1995). A modified TUNEL assay was designed to visualize gaps and DNA fragmentation on metaphase chromosomes. Metaphase chromosomes were prepared as described (Mai, 1994; Mai et al., 1996). Briefly, the slides underwent fixation, RNAse and pepsin treatments, and postfixation. Thereafter, the apoptotic assay was performed using dUTP-fluorescein and the terminal deoxynucleotidyl transferase (TdT) enzyme according to the suppliers (Boehringer Mannheim, Canada). Chromosomes were counterstained with propidium iodide (1 µg/ml) and analysed as described (Mai, 1994; Mai et al., 1996). Chromatin condensation was visualized with DAPI (1 µg/ml). One hundred to three hundred cells were evaluated per sample.

The invention has been described in an illustrative manner, and it is to be understood that the terminology which has been used is intended to be in the nature of words of description rather than of limitation.

Obviously, many modifications and variations of the present invention are possible in light of the above teachings and following Examples. It is, therefore, to be understood that within the scope of the described invention, the invention may be practiced otherwise than as specifically described.

Throughout this application, various publications, including United States patents, are referenced by author and year and patents by number. Full citations for the publications are listed below. The disclosures of these publications and patents in their entireties are hereby incorporated by reference into this application in order to more fully describe the state of the art to which this invention pertains.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the appended claims, the invention may be practiced otherwise than as specifically described.

**Table 1 -**

| CLINICAL FEATURES OF CLL PATIENTS | | | | | | | |
|---|---|---|---|---|---|---|---|
| Patient | Age | Sex | Rai Stage ¹ | Lymphocyte Count (x 10⁹/L) | Doubling Time (Months)² | Time Since Diagnosis (Months) | Prior Treatment³ |
| 1 | 69 | M | 0 | 30 | 13 | 86 | XRay |
| 2 | 61 | F | 0 | 95 | 32 | 44 | - |
| 3 | 66 | F | 0 | 51 | 18 | 101 | - |
| 4 | 72 | F | 0 | 68 | 58 | 96 | - |
| 5 | 67 | F | 0 | 11 | >184 | 184 | - |
| 6 | 55 | F | 0 | 5 | >3 | 3 | - |
| 7 | 71 | M | 1 | 36 | NA | <1 | - |
| 8 | 67 | M | 1 | 14 | >60 | 60 | - |
| 9 | 66 | F | 1 | 64 | 32 | 19 | - |
| 10 | 47 | M | 1 | 29 | - | 66 | Flu |
| 11 | 66 | M | II | 73 | - | 130 | CLB |
| 12 | 81 | F | II | 31 | - | 240 | CLB |
| 13 | 63 | M | II | 56 | - | 41 | Cyclo, Pred |
| 14 | 68 | M | II | 73 | - | 63 | CLB |
| 15 | 66 | M | III | 66 | - | 40 | CLB |
| 16 | 70 | F | III | 57 | - | 96 | CLB, Flu |
| 17 | 66 | M | III | 388 | NA | 1 | - |
| 18 | 78 | F | III | 2 | - | 78 | CLB, Pred |
| 19 | 63 | F | III | | NA | | - |
| 20 | 72 | M | IV | 37 | >5 | 5 | - |
| 21 | 79 | M | IV | 18 | - | 42 | CLB |
| 22 | 71 | M | IV | 126 | - | 180 | CLB, Flu, XRay |
| 23 | 55 | M | IV | 39 | - | 101 | CLB, P, Flu |
| 24 | 73 | M | IV | 15 | - | 45 | CLB |

| | | | | | | | |
|---|---|---|---|---|---|---|---|
| ¹See Table 3 | | | | | | | |
| ² NA, not available. Only applicable in untreated patients | | | | | | | |
| ³ Xray, splenic irradiation; CLB, chlorambucil; Cyclo, cyclophosphamide; P, prednisone, and Flu, fludarabine. | | | | | | | |

**Table 2 -**

| CYCLIN D2 EXPRESSION AND AMPLIFICATION IN CLL PATIENTS | | | | | | | |
|---|---|---|---|---|---|---|---|
| Patient | Protein Level | RNA Level | Gene Amplification | | | | |
| | Western Blot | Northern Blot | FISH | | | | Southern Blot |
| | | | RFI*/Cell | # Cyclin D2 Signals/Cell (%) | | | |
| | | | | ≤ 4 | 4-10 | >10 | |
| Normal B | 1.0 | 1.0 | 1.0 | 100 | | | 1.0 |
| 1 | 1.2 | 1.7 | - | - | - | - | 1.1 |
| 2 | 1.0 | 4.4 | - | - | - | - | 1.1 |
| 3 | 4.5 | 1.8 | - | - | - | - | 1.9 |
| 4 | 3.8 | 0.8 | 24 | | 63 | 37 | 1.3 |
| 5 | - | - | 81 | | | 100 | - |
| 6 | - | - | 12 | | 65 | 35 | - |
| 7 | - | 2.2 | 11 | | 70 | 30 | 1.9 |
| 8 | 3.0 | 2.1 | 6 | | 92 | 8 | - |
| 9 | 2.9 | 3.6 | - | - | - - | - | 2.2 |
| 10 | 2.8 | 3.6 | 7 | | 60 | 40 | - |
| 11 | - | - 4.1 | 42 | | | 100 | 1.6 |
| 12 | 3.2 | 2.4 | - | - | - | - | - |
| 13 | - | 3.9 | 57 | | | 100 | 1.3 |
| 14 | 4.5 | 3.0 | - | - | - | - | 2.2 |
| 15 | - | 4.1 | - | - | - | - | 0.6 |
| 16 | 4.3 | 2.0 | 43 | | | 100 | 1.4 |
| 17 | 6.1 | 2.7 | - | - | - | - | 1.5 |
| 18 | - | - | - | | 80 | 20 | 2.0 |
| 19 | - | 3.6 | - | - | - | - | 1.7 |
| 20 | - | - | - | - | - | - | 2.0 |
| 21 | 5.7 | 3.7 | - | - | - | - | 1.6 |
| 22 | 7.2 | 3.6 | 35 | | 5 | 95 | 1.5 |
| 23 | 4.7 | 2.6 | 14 | | 7 | 93 | 1.5 |
| 24 | - | - | - | | | | 2.6 |

| | | | | | | | |
|---|---|---|---|---|---|---|---|
| *RFI = Relative Fluorescent Intensity/Pixel RFI/Cell = Relative Fluorescent Intensity, i.e., amplification of the *cyclin D'2* gene, per cell | | | | | | | |

### REFERENCES

Mai S., Hanley-Hyde J. and Fluri M. C-Myc overexpression associated DHFR gene amplification in hamster, rat, mouse and human cell lines. Oncogene, 12:277-288 (1996).
Luecke-Huhle C., Mai S. and Moll J. C-myc overexpression facilitates radiation-induced DHFR gene amplification. Int. J. Radiat. Biol., 71:167-175 (1997)
Mai S., Fluri M., Siwarski D. and Huppi, K. Genomic instability in MycER-activated RatlA0MycER cells. Chromosome Res. 4:365-371 (1996).
Fukasawa, K., Wiener, F., Vande Woude, G.F., and Mai, S. Genomic instability and apoptosis are frequent in p53 deficient young mice. Oncogene, 15:1295-1302 (1997).
Inaba, T., Matsushime, Valentine, M., Roussel, M.F., Sherr, C.J. & Look, A.T. Genomic organization, chromosomal localization, and independent expression of human cyclin D genes. Genomics 13:565-574 (1992).
O'Brief S., del Giglio A., Keating M. Advances in the biology and treatment of B-cell chronic lymphocytic leukemia. Blood 85:307-318 (1995).
Rai J.L., Sawitsky A., Cronkite E.P., Chanana A.D., Levy R.N., Pasternack, B.S. Clinical staging of chronic lymphocytic leukemia. Blood 46:219-234 (1975).
Montserrat E., Sanchez-Bisono, J., Vinolas, N. and Rozman, C. Lymphocyte doubling time in chronic lymphocytic leukemia. Analysis of its prognostic significance. Br. J. Haematol. 62:567-575 (1986).
Schena, M., Larsson, L.G., Gottardi, D., et al. Growth-and differentiation- associated expression of bcl-2 in B-chronic lymphocytic leukemia cells. Blood 79:2981-2989 (1992).
Hanada M., Delia, D., Aiello, A., Stadtmauer, E., Reed, J.C. Bcl-2 gene hypomethylation and high-level expression in B-cell chronic lymphocytic leukemia. Blood 82:1820-1828 (1993).
Johnston, J.B., Daeninck, P., Verburg, L., Lee, K., Williams, G., Israels, L.G., Mowat., M.R.A., and Begleiter, A. P53, MDM-2, Bax and Bcl-2 and drug resistance in chronic lymphocytic leukemia. Leuk. Lymph. 26435-449 (1997).
Juliusson, G., Gahrton, G. Chromosome abnormalities in B-cell chronic lymphocytic leukemia. In: Cheson B.D., ed. Chronic lymphocytic leukemia; Scientific advances and clinical developments. Marcel Dekker, Inc. 83-103 (1993)
Juliusson, G., Oscier, D.G., Fitchett, M., et al. Prognostic subgroups in B-cell-chronic lymphocytic leukemia defined by specific chromosome abnormalities. N. Engl. J. Med. 323:720-724 (1990).
Geisler, C., Philip, P., Hansen, M. B-cell chronic lymphocytic leukemia: Clonal chromosome abnormalities and prognosis in 89 cases. Eur. J. Haematol. 43:397-403 (1989).
Oscier, D.G., Stevens, J., Hamblin, T.J., Pickering, T.M., Lambert, R., Fitchett, M. Correlation of chromosome abnormalities with laboratory features and clinical course in B-cell chronic lymphocytic leukaemia. Br. J. Haematol. 76:352-358 (1990).
Kay, N.E., Ranheim, E.A., Peterson, L.C. Tumor suppressor genes and clonal evolution in B-CLL. Leuk. Lymph. 8:416. Kay, N.E., Ranheim, E.A., Peterson, L.C. Tumor suppressor genes and clonal evolution in B-CLL. Leuk. Lymph. 8:41-49 (1995).
Oscier, D., Fitchett, M. Herbert, T., Lambert, R. Karyotypic evolution in B-cell chronic lymphocytic leukaemia. Genes, Chromosomes and Cancer 3:16-20 (1991).
Peterson, L., Blackstadt, M., Kay, N. Clonal evolution in chronic lymphocytic leukemia. In: Cheson BD, ed. Chronic lymphocytic leukemai; Scientific advances and clinical developments. Marcel Dekker, Inc. 181-196 (1993).
Crossen, P.D. Genes and chromosomes in chronic B-cell leukemia. Cancer Genetics Cytogenetics 94:45-51 (1997).
Dohner, H. et al. 11q deletions identify a new subset of B-cell chronic lymphocytic leukemia characterized by extensive nodal involvement and inferior prognosis. Blood 89:2516-2522 (1997)
Corcoran, M.M., Rasool, I., Liu, Y. et al. Detailed molecular delineation of 13q14.3 loss in B-cell chronic lymphocytic leukemia. Blood. 91:1382-1390 (1998).
Kalachikov, Migliazza, Cayanis, E., et al. Clonging and gene mapping of the chromosome 13q14 region deleted in chronic lymphocytic leukemia. Genomics, 42:369-377 (1997).
Matutes, E., et al. Trisomy 12 defines a group of CLL with atypical morphology: correlation between cytogenetic, clinical and laboratory features in 544 patients. Br. J. Haematol, 92:382-388 (1996).
El Rouby, S., et al. p53 gene mutation in B-cell chronic lymphocytic leukemia is associated with drug resistance and is independent of MDR1/MDR3 gene expression. Blood 82:3452-3459 (1993).
Dohner, H., et al. p53 gene deletion predicts for poor survival therapy with purine analogs in chronic B-cell leukemias. Blood 85:1580-1589 (1995).
Garcia-Marco J.A., Caldas, C., Price C.M., Weidemann, L.M., Ashworth, A., Catovsky, D. Frequent somatic deletion of the 13q12.3 locus encompassing BRCA2 in chronic lymphocytic leukemia. Blood 88:1568-1575 (1996).
Santelli, R.V., Machando-Santelli, G.M., Peuyo, M.T., Navarro-Cattapan, L.D. and Lara F.J.S. Replication and transcription in the course of DNA amplfiication of the C3 and C8 puffs of Rhynchosciara americana. Mech. Dev. 36:59-66 (1991).
Delikadis, C. and Kafatos, F.C. Amplification enhancers and replication origins in the autosomal chorion cluster of Drosophila. The Embo. J. 8:891-901 (1989).
Stark, Y. and Wahl, G.M. Gene amplification. Ann. Rev. Biochem. 53:447-491 (1984).
Prody, C.A., Dreyfus, P., Zamir, R., Zakut, H. and Soreq, H. De novo amplification within a 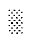 silent 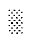 human cholinesterase gene in a family subjected to prolonged exposure to organophosphorus insecticides. Proc. Natl. Acad. Sci. USA 86:690-694 (1989).
Lucke-Huhle, C. Review: Gene amplification - a cellular response to genotoxic stress. Mol. Toxicol. 2:237-253 (1989).
Wright, J.A., Smith, H.S., Watt, F.M., Hancock, M.C., Hudson, D.L., and Stark G.R. DNA amplification is rare in normal human cells. Proc. Natl. Acad. Sci. USA 87:1791-1795 (1990).
Tlsty, T.D. Normal diploid cells lack a detectable frequency of gene amplification. Proc. Natl. Acad. Sci. USA 87:3132-3136 (1990).
Stark, G.R., Regulation and mechanisms of mammalian gene amplification. Adv. Cancer Res. 61:87-113 (1993).
Huang, A., Jin, H., and Wright, J.A. Aberrant expression of basic fibroblast growth factor in NIH-3T3 cells alters drug resistance and gene amplification potential. Exp. Cell. Res. 213:335-339 (1994).
Huang, A., and Wright, J.A. Fibroblast growth factor mediated alterations in drug resistance and evidence of gene amplification. Oncogene 9:491-199 (1994).
Shah, D.M., Horsch, R.B., Klee, H.J. Kishore, G.M. Winter, J.A., Tumer, N.E., Hironaka, C.M., Sanders, P.R., Gasser, C.S., Aykent, S., Siegel, N.R., Rogers, S.G., and Fraley, R.T. Engineering herbicide tolerance in transgenic plants. Science 233:478-481 (1986).
Lucke-Huhle, C. Review: Gene amplification - a cellular response to genotoxic stress. Mol. Toxicol. 2:237-253 (1989).
Lucke-Huhle, C., Pech, M., and Herrlich, P. SV40 CAN amplification and reintegration in surviving hamster cells after 60 Co gamma-irradiation. Int. J. Radiat. Biol. 58:577-588 (1990).
Yalkinoglu, A.O., Zentgraf, H. and Hubscher, U. The origin of adeno-associated virus DNA replication is a target for acrcinogen-induced DNA amplification. J. Virol. 65:3175-3184 (1991).
Mai, S. Overexpression of c-myc precedes amplification of the gene encoding dihydrofolate reductase. Gene 148:253-260 (1994).
Denis, N., Kitzis, A., Kruh, J., Dautry, F., and Crocos, D. Stimulation of methotrexate resistance and dihydrofolate reductase gene amplification by c-myc. Oncogene 6:145301457 (1991).
Johnston, R.N., Beverley, S.M. and Schmike, R.T. Rapid spontaneous dihydrofolate reductase gene amplification shown by fluorescence-activated cell sorting. Proc. Natl. Acad. Sci USA 80:3711-3716 (1983).
Yin, Y., Tainsky, M.A., Bischoff, F.Z., Strong, L.C. and Wahl, G.M. Wildtype p53 restores cell cycle control and inhibits gene amplification in cells with mutant p53 alleles. Cell 70:937-948 (1992).
Livingstone, L.R., White A., Sprouse, J., Livanos, E., Jacks, T., and Tlsty, T.D. Altered cell cycle arrest and gene amplification potential accompany loss of wildtype p53. Cell 70:923-935 (1992).
Van Der Bliek, A.M., Van Der Velde-Koerts, T., Ling, V., Borst, P. Overexpression and amplification of five genes in a multidrug-resistant Chinese hamster ovary line. Mol. Cell. Biol. 6:1671-1678 (1986).
Zhou, P., Jiang, W., Wergost, C.M., Weinstein, I.B., Overexpression of cyclin D1 enhances gene amplification. Cancer Res. 56:36-39 (1996).
Schwab, M. and Amler, L.C. Amplification of cellular oncogenes. A predictor of clinical outcome in human cancer. Genes Chromosomes and Cancer. 1:181-193 (1990).
Hahn, P.J. Molecular biology of couble minute chromosomes. BioEssays. 15:477-484 (1993).
Stark, G.R., Debatisse, M., Giulotto, E., and Wahl, G.M. Recent progress in understanding mechanisms of mammalian DNA amplification. Cell. 57:901-908 (1989).
Carroll, S.M., DeRose, M.L., Gaudray, P., Moore, C.M. Needham-Vandevanter, D.R., Von Hoff, and Wahl, G. Double minute chromosomes can be produced from precursors derived from a chromosomal deletion. Mol. Cell. Biol. 8:1525-1533 (1988).
Windle, B., Draper, B.W., Yin, Y, O'Gorman, S. and Wahl, G.M. A central role for chromosomes breakage in gene amplification, deletion formation, and amplicon integration. Genes Dev. 5:160-174 (1991).
Hamkalo, B.a. Farmham, P.J., Johnston, R., and Schimke, R.T. Ultrastructural features of minute chromosomes in a methotrexate-resistant mouse 3T3 cell line. Proc. Natl. Acad. Sci. USA 82:1126-1130 (1985).
Esnault, C., Lee, H. and Lai, E. Structure and organization of a stable extrachromosomal element in human cells. Gene. 144:205-211 (1994).
Nonet, G.H., Carroll, S.M., DeRose, M.L., and Wahl, G.M. Molecular dissection of an extrachromosomal amplicon reveals a circular structure consisting of an imperfect inverted duplication. Genomics. 15:543-558 (1993).
Sen, S., Sen., P., Mulac-Jericevic, B., Zhou, H. Pirrotta, V., and Stass, S.A. Microdissected double-minute DNA detects variable patterns of chromosomal localizations and multiple abundantly expressed transcripts in normal and leukemic cells. Genomics, 19:542-551 (1994)
Schneider, S.S., Heimstra, J.L., Zehnbauer, B.A., Taillon-Miller, P., Le Paslier, D.L., Vogelstein, B., and Brodeur, G.M. Isolation and structural analysis of a 1.2-megabase N-myc amplicon from a human neuroblastoma. Mol. Cell. Biol. 12:5563-5570 (1992).
Cohen, S., Regev, A., Lavi, S. Induction of circles of heterogeneous sizes in carcinogen-treated cells: Two dimensional gel analysis of circular DNA molecules. Mol. Cell Biol., 16:2002-2014 (1996).
Cohen, S., Regev, A., Lavi, S. Small poorly dispersed circular DNA (spc DNA) in human cells: Associated with genomic instability. Oncogene. 14:977-985 (1997).
Bentz, M., Huck, K. du Manior, S., Joos, S., Werner, D.A., Fischer, K. Dohner, H., and Lichter, H. Comparative genomic hybridization in chronic B-cell leukemias shows a high incidence of chromosomal gains and losses. Blood. 85:3610-3618 (1995).
Merup, M., Juliusson, G., Wu, X., Jansson, M. Stellan, B., Rascool, O., Roijer, E., Stenman, G., Gahrton, G., and Einhorn, S. Amplification of multiple regions of chromosome 12, including 12q13-15, in chronic lymphocytic leukemia. Eur. J. Haematol, 58:174-180 (1997).
Wang, T., Samples, D.M., Doub, R., and Prakash, O. c-myc and K-ras-2 oncogenes in B cell chronic lymphocytic leukemia with del (12(P13)). Cancer Gent. Cytogenet, 51:125-130 (1991).
Greil, R., Fasching, B., Loidl, P. and Huber H. Expression of the c-myc protooncogene in multiple myeloma nad chronic lymphocytic leukemia: An *in situ* analyssi. Blood. 78:180-191 (1991).
Sherr, C.J. G1 phase progression: Cycling on due. Cell, 79:551-555 (1994).
Hirama, T., Koeffler, H.P. Role of the cyclin-dependent kinase inhibitors in the development of cancer. Blood, 86:841-854 (1995).
Delmer, A. Ajchenbaum-Cymbalista, F., Tang, R., Raymond, S., Faussat, A-M., Marie, J-P and Zittoun, R. et al. Overexpression of cyclin Dw in chronic B-cell malignancies. Blood. 85:2870-2876 (1995).
Byrd, J.C., Shimm, C.A. Bedi, A., Waselanko, J.K. Fuchs, E., Flinn, IW, Diehl, L.F., Sausville, E., and Grever, MR> Flavopiridol has marked in vitro activity against human B-chronic lymphocytic leukemia and induces apoptosis independent of p53 status. Blood, 90:401a (1997).
Ando, K, Ajchenbaum-Cymbalista, F and Griffin JD. Regulation of G1/S transition by cyclins D2 and D3 in hematopoietic cells. Proc. Natl. Acad. Sci. (USA), 90: 9571-9575. 1993.
Kato, J-Y, and Sherr, CJ Inhibition of granulocyte differentiation by G1 cyclins D2 and D3 but not D1. Proc Natl Acad Sci (USA), 90: 11513-11517, 1993.
Vrhovac, R, Delmer, A, Tang, JP, Marie, JP, Zittoun, R and Ajchenbaum-Cymbalista, F. The expression of cell cycle inhibitor p27^{kip1} has a prognostic significance and influences apoptosis in B cell chronic lymphocytic leukemia. Blood, 90:91a, 1997.
Muller, D, Bouchard, C, Rudolph, B, Steiner, P, Stuckmann, I, Saffrich, R, Ansorage, W, Huttner, W and Eilers, M. CDK2 dependent phosphorylation of p27 facilitates its Myc-induced release from cyclin E/CDK2 complexes. Oncogene, 15:2561-2576, 1997.
Blain, SW, Montalvo, E, Massague, J. Differential interaction of the cyclin-dependent kinase (CDK) inhibitor p27^{kip1} with cyclinA-Cdk2 and cyclinD2-cdk4. J Biol Chem, 272:25863-25872, 1997.
Kawamata, S, Sakaida, H, Hori, T, Maeda, M and Uchiyama, T. The upregulation of p27^{kip1} by Rapamycin results in G1 arrest in exponentially growing T-cell lines. Blood, 91:561-569, 1998.
Wang, X, Gorospe, M, Huang, Y and Holbrook, NJ. p27^{kip1} overexpression causes apoptotic death of mammalian cells. Oncogene, 15:2991-2997, 1997.
Hoglund, M, Johansson, B, Pedersen-Bjergaard, J, Marynen, P and Mitelman, F. Molecular characterization of 12p abnormalities in hematological malignancies: Deletion of KIP1, rearrangement of TEL, and amplification of CCND2. Bloo, 87:324-330, 1996.
Taylor, C, and Mai, S. C-Myc associated genomic instability of the DHFR locus *in vivo.* Cancer Detection and Prevention 1998. (in press).
Hirt B: Selective extraction of polyoma DNA from infected mouse cell cultures. J Mol Biol, 26:365, 1967.
De Cremous, P, Thious, M, Peter, M, Vielh, P, Michon, J, Delattre, O and Magdelenat, H. Polymerase chain reaction compared with dot blotting for the determination of N-myc gene amplification in enuroblastoma. Int J Cancer, 72:518-521, 1997.
Lawrence, JB, Singer, RH and Marselle, LM. Highly localized tracks of specific transcripts within interphase nuclei visualized by *in situ* hybridization. Cell, 57:493-402, 1989.
Wijgerde, M, Grosveld, F and raser, P. Transcription complex stability and chromatin dynamics *in* vivo. Nature, 377:209-213, 1996.
Ashe, HL, Monks, J, Wijgerde, M, Fraser, P and Proudfoot, NJ. Intergenic transcription and transinduction of the human b-globin locus. Genes and Development, 11:2494-2509, 1997.
Lukas, J, Bartkova, LJ, Welcker, M, Petersen, OW, Peters, G, Strauss, M, and Bartek, J. Cyclin D2 is a moderately oscillating nucleoprotein required for G1 phase progression in specific cell types. Oncogene, 10:2125-2134, 1995.
Larsson L-G, Schena M, Carlsson M, Sallstrom J and Nilsson K. Expression of the c-myc protein is down-regulated at the terminal stages during *in vitro* differentiation of B-type chronic lymphocytic leukemia cells. Blood 77:1025-1032, 1991.
Kubbies, M, Schindler, D, Hoehn, H, and Rabinovitch, PS. BrdU-Hoechst flow cytometry reveals regulation of human lymphocyte growth by donor-age-related growth fraction and transition rate. J Cell Physiol, 125:229-234, 1985.
Schindler, D, Kubbies, M, Hoehn, H, Schinzel, A and Rabinovitch, PS. Confirmation of Fanconi's anemia and detection of a chromosomal aberration (1Q12-32 triplication) via BrdU/Hoechst flow cytometry. Am J Ped Hematol Oncol, 9:172-177, 1987.
Anazodo, MI, Duta, E, Friesen, AD and Wright, JA. Relative levels of inhibition of p24 gene expression by different 20-mer antisense oligonucleotide sequences targeting nucleotides +1129 to +1268 of the HIV-1 gag genome: An analysis of mechanism. Bioch Biophys Res Comm, 229:305-309, 1996.
Mai S and Zjalava A. C-Myc binds to 5' flanking sequence motifs of the dihydrofolate reducatase gene in cellular extracts: Role in proliferation. Nucl Acid Res. 22:2264-2273. 1994.
Fry, CJ, Slansky, JE and Farnham, PJ. Position-dependent transcriptional regulationof the murine dihydrofolate reductase promoter by the E2F transactivation domain. Mol Cell Biol, 17:1966-1976, 1997.
Zheng, CY, Pabello, P, Maksymiuk, AW and Skinnider, LF. Establishment of cell lines derived from chronic lymphocytic leukaemic cells by transfection with my cand ras. Br J Haematol, 93:681-683, 1996.
Harlow, E and Lane, D. 1988. Antibodies: A Laboratory Manual. Cold Spring Harbor Laboratory, Cold Spring Harbor.
Jun, DY, Kim, M-K, Kim, I-G and Kim, YH. Characterization of the murine cyclin D2 gene. Exon/intron organization and promoter activity. Mol Cells, 7:537-543, 1997.
Brooks, AR, Shiffman, D, Chan, CS, Brooks, EE, and Milner, PG. Functional analysis of the human cyclin D2 and cyclin D3 promoters. J Biol Chemistry, 271:9090-9099, 1996.
Reynisdottir, I, Massague, J. The subcellular locations of p15(Ink4b) and p27 ^{(kip1)} coordinate their inhibitory interactions with cdk4 and cdk2. Genes Dev, 11:492-503, 1997.
Bosc, DG, Slominski, E, Sichler, C and Litchfield, DW Phophorylation of casein kinse II by p34cdc2. Identification of phosphorylation sites using phosphorylation site mutants *in vitro.* J Biol Chem, 270:25872-25878, 1995.
Neubauer, A, De Kant, E, Rochlitz, C, Laser, J, Zanetta, AM, Gallardo, J, Oertel, J, Herrmann, R and Huhn, D. Altered expressionof the retinoblastoma susceptibility gene in chronic lymphocytic leukaemia. Br J Haematol, 85:498-503, 1991.
Kornblau, SM, Chen, N, del Giglio, A, O'Brien, S and Deisseroth, AB. Retinoblastoma protein expression is frequently altered in chronic lymphocytic leukemia. Cancer Res, 54:242-246, 1994.
Ganter, B, Fu, S and Lipsick, JS. D-type cyclins repress transcriptional activation by the v-Myb but not the c-Myb DNA-binding domain. The EMBO J., 17:255-268. 1998.
Rosenber, N and Baltimore, D. A quantitative assay for transformation of bone marrow cells by Abelson Murine Leukemia Virus. J Exp Med, 143:1453-1463, 1976.
Graham, FL and Prevec, L. Manipulation of adenovirus vectors. Methods in Mol Biol, 7:109-128, 1991.
Santelli, R.V., Machado-Santelli, G.M., Pueyo, M.T., Navarro-Cattapan, L.d., and Lara, F.J.S. (1991). Replication and transcription in the course of DNA amplification of the C3 and C8 puffs of Rhynochosciara americana. Mech. Dev. 36: 59-66, 1991.
Delikadis, C. and Kafatos, F.C. (1989). Amplification enhancers and replication origins in the autosomal chorion cluster of Drosophila. The EMBO J. 8: 891-901.
Stark, G.R. and Wahl, G.M. (1984). Gene amplification. Ann Rev. Biochem., 53, 447-491.
Yokota, Y., Tsunetsugu-Yokota, Y., Battifora, C.L., and Cline, M.J. (1986). Alterations in myc, myb, ras Ha proto-oncogenes in cancers are frequent and show clinical correlation. Science 231: 261-265.
Mai, S., Hanley-Hyde, J., Fluri, M. (1966). c-Myc overexpression associated DHFR gene amplification in hamster, rat, mouse and human cell lines. Oncogene 12: 277-288.
Mai, S., Fluri, J., Siwarski, D., Huppi, K. (1996). Genomic instability in MycER activated Rat1A-MycER cells. Chromosome Research 4: 365-372.
Mai, S., Hanley-Hyde, J., Coleman, A., Siwarski, D., Huppi, K. (1995). Amplified extrachromosomal elements containing c-Myc and Pvt 1 in a mouse plasmacytoma. Genome 38: 780-85.
Van Der Bliek, A.M., Van Der Velde-Koerts, T., Ling, V., Borst, P. (1986). Overexpression and amplification of five genes in a multidrug-resistant Chinese hamster ovary line. Mol. Cell. Biol. 6: 1671-1678.
Corvi, R., Amler, L. C., Savelyeva, L., Gehring, M., Schwab, M. (1994). MycN is retained in single copy at chromosome 2 band p23-23 during amplification in human neuroblastoma. Proc. Natl. Acad. Sci. (USA) 91: 5523-5527.
Stark, G.R. (1993). Regulation and mechanisms of mammalian gene amplification. Adv. Cancer Res. 61: 87-113.
Schimke, R.T., Kaufman, R.J., Alt, F.W., and Kellems, R.F. (1978). Gene amplification and drug resistance in cultured murine cells. Science 202: 1051-1055.
Shah, D.M., Horsch, R.B., Klee, H.J., Kishore, G.M., Winter, J.A., Tumer, N.E., Hironaka, C.M., Sanders, P.R., Gasser, CS., Aykent, S., Siegel, N.R., Rogers, S.G.., and Fraley, R.T. (1986). Engineering herbicide tolerance in transgenic plants. Science 233: 478-481.
Huang, A. and Wright, J.A. (1994). Fibroblast growth factor mediated alterations in drug resistance, and evidence of gene amplification Oncogene 9: 491-499.
Huang, A., Jin, H., and Wright, J.A. (1994). Aberrant expression of basic fibroblast growth factor in NIH-3T3 cells alters drug resistance and gene amplification potential. Exp. Cell Res. 213: 335-339.
Huang, A., Jin, H., and Wright, J.A. (1995). Drug resistance and gene amplification potential regulated by transforming growth factor-β 1 gene expression. Cancer Res. 55: 1758-1762.
Lavi, S. (1981). Carcinogen-mediated amplification of viral DNA sequences in simian virus 40-transformed Chinese hamster embryo cells. Proc. Natl. Acad. Sci. (USA) 78: 6144-6148.
Tlsty, T.D., Brown, P.E., and Schimke, R.T. (1984). UV radiation facilitates methotrexate resistance and amplification of the dihydrofolate reductase gene in cultured mouse cells. Mol. Cell. Biol. 4 1050-1056.
Lücke-Huhle, C., Pech, M., and Herrlich, P. (1990). SV40 DNA amplification and reintegration in surviving hamster cells after 60 Co gamma-irradiation. Int. J. Radiat. Biol. 58: 577-588.
Yalkinoglu, A.Ö., Zentgraf, H., and Hübscher, U. (1991). The origin of adeno-associated virus DNA replication is a target for carcinogen-induced DNA amplification. J. Virol. 65: 3175-3184.
Lücke-Huhle, C., (1989). Review: gene amplification - a cellular response to genotoxic stress. Mol. Toxicol. 2: 237-253.
Mai, S. (1994). Overexpression of c-myc precedes amplification of the gene encoding dihydrofolate reductase. Gene 148: 253-260.
Denis, N., Kitzis, A., Kruh, J., Dautry, F., and Crocos, D. (1991). Stimulation of methotrexate resistance and dihydrofolate reductase gene amplification by c-myc. Oncogene 6: 1453-1457.
Johnston, R.N., Beverley, S.M., and Schimke, R.T. (1983). Rapid spontaneous dihydrofolate reductase gene amplification shown by fluorescence-activated cell sorting. Proc. Natl. Acad. Sci. (USA) 80: 3711-3715.
Prody, C.A., Dreyfus, P., Zamir, R., Zakut, H., and Soreq, H. (1989). De novo amplification within a 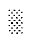 silent 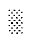 human cholinesterase gene in a family subjected to prolonged exposure to organophosphorus insecticides. Proc. Natl. Acad. Sci. (USA) 86: 690-694.
Wright, J.A., Smith, H.S., Watt, F.M. Hancock, M.C., Hudson, D.L., and Stark, G.R., (1990). DNA amplification is rare in normal human cells. Proc. Natl. Acad. Sci. (USA) 87: 1791-1795.
Tlsty, T.D. (1990). Normal diploid cells lack a detectable frequency of gene amplification. Proc. Natl. Acad. Sci. (USA) 87: 3132-3136, 1990.
Yin, Y., Tainsky, M.A., Bischoff, F.Z., Strong, L.C., and Wahl, G.M. (1992). Wildtype p53 restores cell cycle control and inhibits gene amplification in cells with mutant p53 alleles. Cell 70: 937-948.
Livingstone, L.R., White, A., Sprouse, J., Livanos, E., Jacks, T., and Tlsty, T.D. (1992). Altered cell cycle arrest and gene amplification potential accompany loss of wildtype p53. Cell 70: 923-935.
Zhou, P., Jiang, W., Wegorst, C.M., and Weinstein, I.B. (1996). Overexpression of cyclin D1 enhances gene amplification. Cancer Research 56:36-39.
Marcu, K.B., Bossone, S.A., and Patel, A.J. (1992). Myc function and regulation. Ann. Rev. Biochem. 61: 809-860.
Cole, M.D. (1986). The myc oncogene: its role in transformation and differentiation. Ann. Rev. Genet. 20: 361-384.
Benevisty, N., Leder, A., Kuo, A., and Leder, P. (1992). An enbryonically expressed gene is a target for c-Myc regulation via the c-Myc binding sequence. Genes Dev. 6: 2513-2523.
Bello-Fernandez, C., Packham, G., and Cleveland, J.L. (1993). The ornithin decarboxylase is a transcriptional target of c-Myc. Proc. Natl. Acad. Sci. (USA) 90: 7804-7808.
Gaubatz, S., Meichle, A., and Eilers, M. (1994). An E-box element localized in the first intron mediates regulation of the prothymosin a gene by c-myc. Mol. Cell. Biol. 14: 3853-3862.
Jansen-Dürr, P., Meichle, A., Steiner, P., Pagano, M., Finke, K., Botz, J., Wessbecher, J., Draetta, G., and Eilers, M. (1993). Differential modulation of cyclin expression by MYC. Proc. Natl. Acad. Sci. (USA) 90: 3685-3689.
Daksis, J.I., Lu, R.Y., Facchini, L.M., Marhin, W.W., and Penn, L.J.Z. (1994). Myc induces cyclin D1 expression in the absence of de novo protein synthesis and links mitogen-stimulated signal transduction to the cell cycle. Oncogene 9: 3635-3645, 1994.
Philipp, A., Schneider, A., Västrik, I. Finke, K., Xiong, Y., Beach, D., Alitalo, K., and Eilers, M. (1994). Repression of cyclin D1: a novel function of MYC. Mol. Cell. Biol. 14: 4032-4043.
Galaktionov, K., Chen, X., and Beach, D. (1996). Cdc25 cell-cycle phosphatase as a target of c-myc. Nature 382: 511-517.
Roy, A.L., Carruthers, C., Gutjahr, T., and Roeder, R.G. (1993). Direct role for Myc in transcription initiation mediated by interactions with TFII-I. Nature 365: 359-361.
Li, L. -h., Nerlov, C., Prendergast, G., MacGregor, D., and Ziff, E.B. (1994). c-Myc represses transcription *in vivo* by a novel mechanism dependent on the initiator element and Myc box II. The EMBO J. 13: 4070-4079.
Mai, S. and Martensoon, I.-L. (1995). The c-myc protein represses λ 5 and TdT initiators. Nucl. Ac. Res. 23: 1-9.
Heikkila, R., Schwab, G., Wickstrom, E., Loke, S.L., Pluznik, D.H., Watt, R., and Neckers, L.M. (1987). A c-myc antisense oligodeoxynucleotide inhibits entry into S phase but not pgoress from Go to G1. Nature 328: 445-449.
Karn, J., Watson, J.V., Lowe, A.D., Green, S.M., and Vedeckis, W. (1989). Regulation of cell cycle duration by c-myc levels. Oncogene 4: 773-787.
Hanson, K.D., Schichiri, M., Follansbee, J.R., and Sedivy, J.M. (1994). Effects of c-myc expression on cell cycle progression. Mol. Cell. Biol. 14: 5748-5755.
Stanton, L.W., Watt, R., Marcu, K.B. (1983). Translocation, breakage, and truncated transcripts of c-myc oncogene in murine plasmacytomas. Nature 303: 401-406.
Potter, M. and Wienr, F. (1992). Plasmacytomagenesis in mice: model of neoplastic development dependent upon chromosomal translocation. Carcinogenesis 13: 1681-1697.
Feo, S., Liegro, C.D., Jones, T., Read, M., and Fried, M. (1994). The DNA region around the c-myc gene and its amplification in human tumour cell lines. Oncogene 9: 955-961.
Alito, K. (1985). Amplification of cellular oncogenes in cancer cells. TIBS 10: 194-197.
Classon, M., Henriksson, M., Klein, G., and Hammaskjold, M.-L. (1987). Elevated c-myc expression facilitates the replication of SV40 in human lymphoid cells. Nature 330: 272-274.
Classon, M., Henriksson, M., Klein, G. and Hammerskjold, M.-L. (1990). The effect of c-myc protein on SV40 replication in human lymphoid cells. Oncogene 5: 1371-1376.
Classon, M., Wennborg, M., Klein, G., and Sumegi, J. (1993). Analysis of c-Myc domains involved in stimulating SV40 replication. Gene 133: 153-161.
Luecke-Huhle, C., Mai, S., Herrlich, P. (1989). UV-inducible early-domain binding factor as the limiting component of Simian Virus 40 DNA amplification in rodent cells. Mol. Cell. Biol. 9: 4812-4818.
Mai, S., Lücke-Huhle, C., Kaina, B., Rahmsdorf, H.J., Stein, B., Ponta, H., and Herrlich, P. (1990):Ionizing radiation induced formation of a replication origin binding complex involving the product of the cellular oncogene c-Myc. In: Ionizing Radiation Damage of DNA. Molecular Aspects. Wiley-Liss., New York, NY, 319-331.
Mai, S. and Jalava, A. (1994). c-Myc binds to 5' flanking sequence motifs of the dihydrofolate reductase gene in cellular extracts: role in proliferation. Nucl. Acids Res. 22: 2264-2273.
Wells, J., Held, P., Illenye, S., and Heintz, N.H. (1996). Protein-DNA interactions at the major and minor promoters of the divergently transcribed *dhfr* and *rep* 3 genes during the Chinese hamster ovary cell cycle. Mol. Cell. Biol. 16: 634-647.
Luecke-Huhle, C., Mai, S., Moll, J. (1996). Correlation of gene expression and gene amplification. Proc. of the ICRR pp. 560-564.
Kunz, B.A., Kohalmi, S.E., Kunkel, T.A., Mathews, C.K., Mcintosh, E.M., Reidy, J.A. (1994). Deoxyribonucleoside triphosophate levels: a critical factor in the maintenance of genetic stability. Mut. Res. 318: 1-64.
Luecke-Huhle, C. (1994). Permissivity for methotrexate-induced DHFR gene amplification correlates with the metastic potential of rat adenocarcinoma cells. Carcinogenesis 15: 695-700.
Mai, S. and Jalava, A. 1994. c-Myc binds to 5' flanking sequence motifs of the dihydrofolate reductase gene in cellular extracts: role in proliferation. *Nucl. Acids Res.* 22: 2264-2273.
Denis, J., Kitzis, A., Kruh, J., Dautry, F., and Crocos, D. 1991. Stimulation of methotrexate resistance and dihydrofolate reductase gene amplification by c-myc. *Oncogene* 6: 1453-1457.
Wells, J., Held, P., Illenye, S., and Heintz, N.H. 1996. Protein-DNA interactions at the major and minor promoters of the divergently transcribed *dhfr* and *rep 3* genes during the Chinese hamster ovary cell cycle. Mol. Cell. Biol 16: 634-647.
Mai, S. 1994. Overexpression of c-myc precedes amplification of the gene encoding dihydrofolate reductase. *Gene* 148: 253-260.
Mai, S., Hanley-Hyde, J., Fluri, M. 1996. c-Myc overexpression associated DHFR gene amplification in hamster, rat, mouse and human cell lines. *Oncogene* 12: 277-288.
Luecke-Huhle, C., Mai, S., Moll, J. 1996. Correlation of gene expression and gene amplification. *Proc. of the ICRR.* pp. 560-564.
Mai, s., Fluri, M., Siwarski, D., Huppi, K. 1996. Genomic instability in MycER activated RatlA-MycER cells. *Chromosome Research* 4: 1-7.
Fukasawa, K., Wiener, F., Vande Woude, G.f., Mai, S. 1997. Genomic instability and apoptosis are frequent in p53 deficient young mic. *Oncogene* 15: 1295-1302.
Potter and Wiener, 1992. Plasmacytomagenesis in mice: model of neoplastic development dependent upon chromosomal translocation. *Carcinogenesis* 13: 1681-1697.
Mock, B., Krall, M.M., and Dosik, J.K. 1993. Genetic mapping of tumor susceptibility genes in mouse plasmacytomagenesis. *Proc. Natl. Acad. Sci. (USA)* 90: 9499-9503.
Potter, M. Mushinski, E.B., Wax, J.S., Hartley, J., and Mock, B.A. 1994. Identification of two genes on chromosome 4 that determine resistance to plasmacytoma induction in mice. *Cancer Research* 54: 969-975.
Silva, S., Wang, Y., Babonits, M., Imreh, S., Wiener, F., Klein, G. 1997. Spontaneous development of plasmacytomas in a selected subline of Balbc/cJ mice. *Eur. J. Cancer* 33: 479-485.
Evan, G.I., Lewis, G.K., Ramsay, G., Bishop, J.M. 1985. Isolation of monoclanal antibodies specific for human c-myc proto-oncogene product. *Mol. Cell. Biol.* 5: 3610-3616.
Chang, A.C., Nunberg, J.H., Kaufman, R.J., Erlich, H.A., Schimke, R.T., Cohen, S.N. 1978. Phenotypic expression in E. coli of a DNA sequence coding for mouse dihydrofolate reductase. *Nature* 275: 617-624.
Eckschlager, T. and McClain, K. 1996. Comparison of fluorescent *in situ* hybridization (FISH) and the polymerase chain reaction (PCR) for detection of residual neuroblastoma cells. *Neoplasma* 43: 301-303.
White, D.L, Hutchins, C.J., Turczynowicz, S., Suttle, J., Haylock, D.N., Hughes, T.P., Juttner, C.A., To, L.B. 1997. Detection of minimal residual disease in an aml patient with trisomy 8 using interphase FISH. *Pathology* 29: 289-293.
Afify, A. and Mark, H.F. 1997. Fluorescence *in situ* hybridization assessment of chromosome 8 copy number in stage I and stage II infiltrating ductual carcinoma of the breast. Cancer Genet. *Cytogenet.* 97 : 101 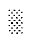 105.
Kunz, J.A. 1994. Deoxyribonucleoside triphosphate levels: a critical factor in the maintenance of genetic stability. *Mut. Res.* 318: 1-64.
Luecke-Huhle, C. 1994. Permissivity for methotrexate-induced DHFR gene amplification correlates with the metastic potential of rat adenocarcinoma cells. *Carcinogenesis* 15: 695-700.
Burke and Olson, "Preparation of Clone Libraries in Yeast Artificial-Chromosome Vectors" in Methods in Enzymology, Vol. 194, "Guide to Yeast Genetics and Molecular Biology", eds. C. Guthrie and G. Fink, Academic Press, Inc., Chap. 17, pp. 251-270 (1991).
Capecchi, "Altering the genome by homologous recombination" Science 244:1288-1292 (1989).
Davies et al., "Targeted alterations in yeast artificial chromosomes for inter-species gene transfer", Nucleic Acids Research, Vol. 20, No. 11, pp. 2693-2698 (1992).
Dickinson et al., "High frequency gene targeting using insertional vectors", Human Molecular Genetics, Vol. 2, No. 8, pp. 1299-1302 (1993).
Duff and Lincoln, "Insertion of a pathogenic mutation into a yeast artificial chromosome containing the human APP gene and expression in ES cells", Research Advances in Alzheimer's Disease and Related Disorders, 1995.
Huxley et al., "The human HPRT gene on a yeast artificial chromosome is functional when transferred to mouse cells by cell fusion", Genomics, 9:742-750 (1991).
Jakobovits et al., "Germ-line transmission and expression of a human-derived yeast artificial chromosome", Nature, Vol. 362, pp. 255-261 (1993).
Lamb et al., "Introduction and expression of the 400 kilobase *precursor amyloid protein* gene in transgenic mice", Nature Genetics, Vol. 5, pp. 22-29 (1993).
Pearson and Choi, *Expression of the human $-amyloid precursor protein gene from a heast artificial chromosome in transgenic mice.* Proc. Natl. Scad. Sci. USA, 1993. 90:10578-82.
Rothstein, "Targeting, disruption, replacement, and allele rescue: integrative DNA transformation in yeast" in Methods in Enzymology, Vol. 194, "Guide to Yeast Genetics and Molecular Biology", eds. C. Guthrie and G. Fink, Academic Press, Inc., Chap. 19, pp. 281-301 (1991).
Schedl et al., "A yeast artificial chromosome covering the tyrosinase gene confers copy number-dependent expression in transgenic mice", Nature*,* Vol. 362, pp. 258-261 (1993).
Strauss et al., "Germ line transmission of a yeast artificial chromosome spanning the murine "₁ (I) collagen locus", Science, Vol. 259, pp. 1904-1907 (1993).
Gilboa, E, Eglitis, MA, Kantoff, PW, Anderson, WF: Transfer and expression of cloned genes using retroviral vectors. BioTechniques 4(6):504-512, 1986.
Cregg JM, Vedvick TS, Raschke WC: Recent Advances in the Expression of Foreign Genes in *Pichia pastoris,* Bio/Technology 11:905-910, 1993
Culver, 1998. Site-Directed recombination for repair of mutations in the human ADA gene. (Abstract) Antisense DNA & RNA based therapeutics, February, 1998, Coronado, CA.
Agrawal, 1996. Antisense oligonucleotides: towards clinical trials, TIBTECH, 14:376.
Akhter et al, 1991. Interactions of antisense DNA oligonucleotide analogs with phospholipid membranes (liposomes). Nuc. Res. 19:5551-5559.
Blaesse, 1997. Gene Therapy for Cancer. Scientific American 276(6):111-115.
Calabretta, et al, 1996. Antisense strategies in the treatment of leukemias. Semin. Oncol. 23:78.
Crooke, 1995. Progress in antisense therapeutics, Hematol. Pathol. 2:59.
Felgner, 1997. Nonviral Strategeies for Gene Therapy. Scinetific American. June, 1997, pgs 102-106.
Gewirtz, 1993. Oligodeoxynucleotide-based therapeutics for human leukemias, Stem Cells Dayt. 11:96.
Hanania, et al 1995. Recent advances in the application of gene therapy to human disease. Am. J. Med. 99:537.
Lefebvre-d'Hellencourt et al, 1995. Immunomodulation by cytokine antisense oligonucleotides. Eur. Cytokine Netw. 6:7.
Lev-Lehman et al., 1997. Antisense Oligomers *in vitro* and *in vivo.* In Antisense Therapeutics, A. Cohen and S. Smicek, eds (Plenum Press, New York)
Loke et al, 1989. Characterization of oligonucleotide transport into living cells. PNAS USA 86:3474.
Morrison, 1991. Suppression of basic fibroblast growth factor expression by antisense oligonucleotides inhibits the growth of transformed human astrocytes. J. Biol. Chem. 266:728.
Rosolen et al., 1990. Cancer Res. 50:6316.
Uhlmann and Peyman, 1990. Antisense Oligonucleotides: A New Therapeutic Principle. Chem Rev 90(4):543-584.
Wagner et al., 1996. Potent and selective inhibition of gene expression by an antisense heptanucleotide. Nature Biotechnology 14:840-844.
Wagner, 1994. Gene inhibition using antisense oligodeoxynucleotides. Nature 372:333.
Whitesell et al., 1991. Episome-generated *N-myc* antisense RNA restricts the differentiation potential of primitive neuroectodermal cell lines. Mol. Cell. Biol. 11:1360.
Yakubov et al, 1989. PNAS USA 86:6454.
Wright & Anazodo, 1995. Antisense Molecules and Their Potential For The Treatment Of Cancer and AIDS. Cancer J. 8:185-189.
Scanlon et al., 1995. Oligonucleotides-mediated modulation of mammalian gene expression. FASEB J. 9:1288.
Galileo et al., 1991. J. Cell. Biol., 112:1285. Gewirtz, 1993. Oligodeoxynucleotide-based therapeutics for human leukemias, Stem Cells Dayt. 11:96.
Eckstein 1985. Nucleoside Phosphorothioates. Ann. Rev. Biochem. 54:367-402.
Iyer et al. 1990. J. Org. Chem. 55:4693-4699.
Radhakrishnan et al., 1990. The automated synthesis of sulfur-containing oligodeoxyribonucleotides using 3H-1,2-Benzodithiol-3-One 1,1 Dioxide as a sulfur-transfer reagent. J. Org. Chem. 55:4693-4699.
Shaw et al., 1991. Modified deoxyoligonucleotides stable to exonuclease degradation in serum. Nucleic Acids Res. 19:747-750.
Spitzer and Eckstein 1988. Inhibition of deoxynucleases by phosphorothioate groups in oligodeoxyribonucleotides. Nucleic Acids Res. 18:11691-11704.
Woolf et al., 1990. The stability, toxicity and effectiveness of unmodified and phosphorothioate antisense oligodeoxynucleotides in *Xenopus* oocytes and embryos. Nucleic Acids Res. 18:1763-1769.
Blackwood EM and Eisenman RT. (1991). Science 251, 1211-1216.
Brooks AR, Shiffman D, Chan CS, Brooks EE and Milner PG. (1996). J. Biol. Chem. 271, 9090-9099.
Citri Y, Braun J and Baltimore D. (1987). J. Exp. Med. 165, 1188-1194.
Cohen S, Regev A and Lavi S. (1997). Oncogene 14 977-985. Cole MD. (1986). Ann Rev. Genet. 20, 361-384.
Coleman AE, Schröck E, Weaver Z, du Manoir S, Yang F, Ferguson-Smith MA, Ried T and Janz S. (1997). Can. Res. 57, 4585-4592.
Daksis JI, Lu RY, Facchini LM, Marhin WW and Penn LJZ. (1994). Oncogene 9, 3635-3645.
Erisman, MD, Scott JK, Watt RA and Astrin SM. (1988). Oncogene 2, 367-378.
Fan H, Villegas C and Wright JA. (1996). Proc. Natl. Acad. Sci. USA 93, 14036-14040.
Feo S. Liegro CD, Jones T, Read M and Fried M. (1994). Oncogene 9, 955-961.
Fort P, Marty L, Piechaczyk M, El Sabrouty S, Dani C, Jeanteur P and Blanchard JM. (1985). Nucl. Acids Res. 13, 1431-1437.
Galaktionov K., Chen X and Beach D. (1996). Nature 382, 511-517.
Gurfinkel N, Unger T, Givol D and Mushinski JF. (1987). Eur.J.Immunol. 17, 567-570.
Hamel PA and Hanley-Hyde J. (1997). Cancer Investigation 15, 143-152.
Hanna Z, Jankowski M, Tremblay P, Jiang X, Milatovich A, Francke U and Jolicoeur P. (1993). Oncogene 8, 1661-1666.
Hanson KD, Shichiri M, Follansbee MR and Sedivy JM. (1994). Mol. Cell Biol. 14, 5748-5755.
Hayward WS, Neel BG and Astrin SM. (1981). Nature 290, 475-480.
Heikkila R, Schwab G, Wickstrom E, Loke SL, Pluznik DH, Watt R and Neckers LM (1987). Nature 328, 445-449.
Jaffe BM, Eisen HN, Simms ES and Potter M. (1969). J. Immunol. 103, 872-878.
Jansen-Deurr P, Meichle A, Steiner P, Pagano M, Finke K, Botz J, Wessbecher J, Draetta G and Eilers M. (1993). Proc. Natl. Acad. Sci. USA 90, 3685-3689
Jiang W, Kahn SM, Zhou P, Zhang YJ, Cacace AM, Infante SD, Santella RM and Weinstein IB. (1993). Oncogene 8, 3447-3457.
Jun DY, Kim MK, Kim IG and Kim YH. 1997. Mol. Cells 7, 537-543.
Karn J, Watson JV, Lowe AD, Green SM and Vedeckis W. (1989). Oncogene 4, 773-787.
Kiyokawa H, Busquets X, Powell CT, Ngo L, Rifkind RA and Marks PA. (1992). Proc. Natl. Acad. Sci. USA 89, 2444-2447.
Leach FS, Elledge SJ, Sherr CJ, Willson JKV, Markowitz S, Kinzler KW and Vogelstein B (1993). Cancer Res. 53, 1986-1989.
Littlewood TD, Hancock DC, Danielian PS, Parker MG and Evan GI. (1995). Nucl. Acids Res. 23, 1686-1690.
Luecke-Huhle C. (1994). Carcinogenesis 15, 695-700.
Mai S. (1994). Gene 148, 253-260.
Mai S, Hanley-Hyde J, Coleman A, Siwarski D and Huppi K. (1995). Genome 38, 780-785.
Mai S. Hanley-Hyde J. and Fluri M. 1996. Oncogene 12, 277-288.
Mai S and Jalava A. (1994). Nucl. Acids Res. 22, 2264-2273.
Mann R, Mulligan RC and Baltimore D. (1983). Cell 33, 153-159.
Marcu KB, Bossone SA and Patel AJ. (1992). Ann. Rev. Biochem. 61, 809-860.
Matsushime H, Roussel MF, Ashmun RA and Sherr CJ. (1991). Cell 65, 701-713.
Mischak H, Goodnight J, Kolch W, Martiny-Baron G, Schaechtle C, Kazanietz, MG, Blumberg PM, Pierce JH and Mushinski JF. (1993). J. Biol. Chem. 268, 6090-6096.
Morse B, Rotherg PG, South VJ, Spandorfer JM and Astrin SM. (1993). Nature 333, 87-90.
Motokura T and Arnold A. Curr. Opin. Genet. Dev. 3, 5-10.
Mushinski JF. (1988). In Cellular Oncogene Activation (ed. Klein) pp. 181-211. Marcel Dekker, New York and Basel.
Mushinski JF, Davidson WD and Morse HC. (1987). Cancer Invest. 5, 345-368.
Pear WS, Wahlstrom G, Nelson SF, Axelson H, Szeles A, Wiener F, Bazin H, Klein G and Sumegi J. (1988). Mol. Cell Biol. 8, 441-451.
Philipp A, Schneider A, Vaestrik I, Finke K, Xiong Y, Beach D, Alitalo K and Eilers M. (1994). Mol. Cell Biol. 14, 4032-4043.
Press MF, Bernstein L, Thomas PA, Meisner LF, Zhou J-Y, Ma Y, Hung G, Robinson RA, Harris C. El-Naggar A, Slamon DJ, Phillips RN, Ross JS, Wolman SR and Flom KJ. (1997). J. Clin. Oncol. 15, 2894-2904.
Rosenberg N, and Baltimore D. (1976). J. Exp. Med. 143, 1453-1463.
Sambrook J, Fritsch EF and Maniatis T. (1989). A laboratory manual. Cold Spring Harbor.
Shen-Ong GLC, Keath EJ, Piccoli SP and Cole MD. (1982). Cell 31, 443-480.
Sinclair AJ, Palmero I, Peters G and Farrell PJ. (1994). EMBO J 13, 3321-3328.
Southern EM. (1975). J. Biol. Chem. 253, 5852-5860. Stanton, LW, Watt R and Marcu KB. (1983). Nature 303, 401-406.
Steiner P, Philipp A, Lukas J, Godden-Kent D, Pagano M, Mittnacht S, Bartek J and Eilers M. (1995). EMBO J 14, 4814-4826.
Taub R, Kirsch I, Morton C, Lenoir GM, Swan D, Tronick S, Aaronson S and Leder P. (1982). Proc. Natl. Acad. Sci. USA 79, 7837-7841.
Thelander L and Berg P. (1986). Mol. Cell Biol. 6, 3433-3442.
Wang TC, Cardiff Rd, Zuckerberg L, Lees E, Arnold A and Schmidt EV. (1994). Nature 369, 669-671.
Waters CM, Littlewood TD, Hancock DC, Moore JP and Evan GI. (1991). Oncogene 6, 797-805.
Yokota Y, Tsunetsugu-Yokota Y, Battifora CL and Cline MJ. (1986). Science 231, 261-265.
Zhang S-Y, Liu S-C, Goodrow T, Morris R and Klein-Szanto AJP. (1997). Mol. Carcinogenesis 18, 142-152.
Zhou P, Jiang W Zhang Y, Kahn SM, Schieren I, Santella RM and Weinstein IB. (1995). Oncogene 11, 571-580.

## Claims

1. A method for identifying pre-malignancy and malignancy states of a cell by detecting extrachromosomal gene amplification.

2. The method according to claim 1, wherein said detecting step is further defined as detecting extrachromosomal gene amplification of genes from the group consisting essentially of DHFR, c-Myc, immunoglobulin genes, anti-apoptosis genes, and drug-resistance genes.

3. The method according to claim 1, wherein said detecting step further includes detecting gene amplification using a combined protein and FISH analysis.

4. The method according to claim 1, wherein said detecting step further includes detecting gene amplification using quantitative fluorescence immunohistochemistry.

5. A marker for the identification of pre-malignancy and malignancy states of a cell comprising extrachromosomal gene amplification.

6. The marker according to claim 5, wherein said extrachromosomal gene amplification includes genes from the group consisting essentially of DHFR, c-Myc, immunoglobulin genes, anti-apoptosis genes, and drug-resistance genes.

7. A method of therapeutic intervention in cells having extrachromosomal gene amplification by therapeutically targeting the genes having extrachromosomal amplification.

8. The method according to claim 7, wherein said therapeutically treating step further includes treating with gene therapy.

9. The method according to claim 8, wherein said treating with gene therapy step includes utilizing suicide genes targeted toward extrachromosomal elements.

10. The method according to claim 8, wherein said treating with gene therapy step includes utilizing antisense therapy targeted to the identified gene.

11. A kit for identifying pre-malignancy and malignancy states of a cell, comprising:
detector means for detecting extrachromosomal gene amplification.

12. The kit according to claim 11, wherein said detector means detecting extrachromosomal gene amplification of genes from the group consisting essentially of DHFR, C-MyC, immunoglobulin genes, anti-apoptosis gene and drug-resistance genes.

13. The kit according to claim 11, wherein said detector means including a combined protein and FISH analysis.

14. The kit according to claim 11, wherein the kit further includes a marker for the identification of pre-malignancy and malignancy states of a cell having extrachromosomal gene amplification.

15. The kit according to claim 14, whereir said marker identifies extrachromosomal gene amplification of genes from the group consisting essentially of DHFR, C-Myc, immunoglobulin genes, anti-apoptosis genes and drug-resistance genes.

16. A kit for therapeutic intervention of cells having extrachromosomal gene amplification comprising of a therapeutic targeting means for targeting the genes having extrachromosomal amplification.

17. The kit according to claim 16, wherein said treatment means is gene therapy.

18. The kit according to claim 17, wherein said gene therapy includes antisense therapy targeted to the identified gene.

19. Extrachromosonal gene amplification for use as diagnostic.

20. Means for detecting extrachromosonal gene amplification for use as a medicament or diagnostic.

21. The use of means for detecting extrachromosonal gene amplification in the manufacture of a medicament for treating or diagnosing pre-malignancy and malignant states of a cell.
